# FASCICULE DE BREVET EUROPEEN

(11) **EP 1 414 967 B1**
(45) Date de publication et mention de la délivrance du brevet: **15.04.2015**
(21) Numéro de dépôt: 02751257.3
(22) Date de dépôt: 17.06.2002
(51) Int. Cl.: C12N 15/30, C12N 9/90, C07K 14/44, G01N 33/50, G01N 33/569, G01N 33/573, C07K 16/40, C07K 16/20, C12Q 1/68, A61K 39/00

(54) **GENE ASSOCIE A LA VIRULENCE DU PARASITE LEISHMANIA**
GEN DER MIT DER VIRULENZ DES LEISHMANIA PARASITEN ASSOZIIERT IST
GENE ASSOCIATED WITH LEISHMANIA PARASITE VIRULENCE

(30) Priorité: 18.06.2001 FR 0107985
(43) Date de publication de la demande: 06.05.2004
(73) Titulaire: INSTITUT PASTEUR DE TUNIS, 1002 Tunis Belvedere (TN); INSTITUT PASTEUR, 75724 Paris Cedex 15 (FR)
(72) Inventeur: BEN ACHOUR, Yosser, 2070 La Marsa (TN); CHENIK, Mehdi, 2070 La Marsa (TN); LOUZIR, Hechmi, 2070 La Marsa (TN); DELLAGI, Koussay, 1002 Tunis (TN)
(74) Mandataire: Desaix, Anne
(86) Numéro de dépôt international: PCT/FR2002/002086
(87) Numéro de publication internationale: WO 2002/103002

(56) Documents cités:
- CA-A- 2 105 538
- PADILLA, A. ET AL.: "Isolation of a unique protein-disulfide isomerase gene from Leishmania donovani" -, [en ligne] 15 - 16 février 2001, XP002191794 Extrait de l'Internet: <URL:http://vm.cfsan.fda.gov/~frf/forum01/ A294N12.htm> [extrait le 2002-02-26]
- DATABASE EM_HTG [en ligne] Numéro d'accès : AL499624 (ID : LMFLCHR36), 16 décembre 2000 (2000-12-16) "Leishmania major Friedlin assembled shotgun reads from chromosome 36" XP002191798
- DATABASE EM_EST [en ligne] Numéro d'accès : AA680895, 8 décembre 1997 (1997-12-08) "LmFrAm0479 Leishmania major amastigote full length cDNA library Leishmania major cDNA clone Q52 5'" XP002191799
- MANDAL DEBJANI ET AL: "The plasma-membrane Ca-2+-ATPase of Leishmania donovani is an extrusion pump for Ca-2+." BIOCHEMICAL JOURNAL, vol. 322, no. 1, 1997, pages 251-257, XP002191795 ISSN: 0264-6021
- CLIVE D R ET AL: "ASSOCIATION OF PROTEIN DISULFIDE ISOMERASE ACTIVITY AND THE INDUCTION OF CONTACT INHIBITION" EXPERIMENTAL CELL RESEARCH, vol. 214, no. 1, 1994, pages 139-144, XP000907390 ISSN: 0014-4827
- ABDELHAK S ET AL: "RECOMBINANT BCG EXPRESSING THE LEISHMANIA SURFACE ANTIGEN GP63 INDUCES PROTECTIVE IMMUNITY AGAINST LEISHMANIA MAJOR INFECTION IN IN BALB/C MICE" MICROBIOLOGY, vol. 141, no. 7, 1 juillet 1995 (1995-07-01), pages 1585-1592, XP000560430 ISSN: 1350-0872
- LYLES M M ET AL: "CATALYSIS OF THE OXIDATIVE FOLDING OF RNASE A BY PROTEIN DISULFIDE ISOMERASE DEPENDENCE OF THE RATE ON THE COMPOSITION OF THE REDOX BUFFER" BIOCHEMISTRY, vol. 30, no. 3, 1991, pages 613-619, XP002247186 ISSN: 0006-2960 cité dans la demande
- MOU Y ET AL: "The selective inhibition of beta 1 and beta 7 integrin-mediated lymphocyte adhesion by bacitracin." JOURNAL OF IMMUNOLOGY (BALTIMORE, MD. : 1950) 1 DEC 1998, vol. 161, no. 11, 1 décembre 1998 (1998-12-01), pages 6323-6329, XP002247187 ISSN: 0022-1767 cité dans la demande
- HEARD PAMELA L ET AL: "Leishmania mexicana amazonensis: Differential display analysis and cloning of mRNAs from attenuated and infective forms." JOURNAL OF EUKARYOTIC MICROBIOLOGY, vol. 43, no. 5, 1996, pages 409-415, XP001064031 ISSN: 1066-5234 cité dans la demande
- YU JUN ET AL: "Key role for DsbA in cell-to-cell spread of Shigella flexneri, permitting secretion of Ipa proteins into interepithelial protrusions." INFECTION AND IMMUNITY, vol. 68, no. 11, novembre 2000 (2000-11), pages 6449-6456, XP002191796 ISSN: 0019-9567 cité dans la demande
- LOUZIR, H.: "Novel gene associated with Leishmania parasite virulence: therapeutic and vaccine applications" INSTITUT PASTEUR LICENSING OPPORTUNITY DI 00-80, [en ligne] XP002191816 Extrait de l'Internet: <URL:www.pasteur.fr/applications/dri/Engli sh/DI/00.80.html> [extrait le 2002-02-22]
- KEBAIER C ET AL: "Heterogeneity of wild Leishmania major isolates in experimental murine pathogenicity and specific immune response." INFECTION AND IMMUNITY, vol. 69, no. 8, août 2001 (2001-08), pages 4906-4915, XP002191797 ISSN: 0019-9567 cité dans la demande
- BEN ACHOUR Y ET AL: "Identification of a disulfide isomerase protein of Leishmania major as a putative virulence factor." INFECTION AND IMMUNITY, vol. 70, no. 7, juillet 2002 (2002-07), pages 3576-3585, XP002247188 ISSN: 0019-9567

### LISTE DE SEQUENCES

<110> INSTITUT PASTEUR INSTITUT PASTEUR DE TUNIS
<120> GENE ASSOCIE A LA VIRULENCE DU PARASITE LEISHMANIA
<130> B4866A -AD/VMA
<140>
   <141>
<150> FR 0107985
   <151> 2001-06-18
<160> 10
<170> PatentIn Ver. 2.1
<210> 1
   <211> 2094
   <212> ADN
   <213> Leishmania major
<220>
   <221> CDS
   <222> (241) .. (1674)
   <223> Séquence codante (orf) de la LmPDI
<400> 1
   ggcaccagcg gcaccagagt ttctgtactt tattgtcttt ctctattcta cccacacttg 60
   cctctctgcg ctctctgtgc ctgtgcgggc gcgcaacgtg cctttctctc cgtattgccc 120
   agctgtgagc tgctgcctac tggcaacgtg tacgccattc ccgtttcttg attctggtgc 180
   agtgctcagc tctaccctat ttgtattgat accgttttcc ttttcgtttt gcaaagaaaa 240
   atg cag cgc tca ttc ctt gtt ttt gtt ctg tgc gcc ctt ctc ttc tgc   288
   Met Gln Arg Ser Phe Leu Val Phe Val Leu Cys Ala Leu Leu Phe Cys
    1               5                  10                  15
   gtc gcg tcc gca gag gtg cag gtg gcc act aag gac aac ttt gac aag   336
   Val Ala Ser Ala Glu Val Gln Val Ala Thr Lys Asp Asn Phe Asp Lys
               20                  25                  30
   gtc gta atc ggg gat ctc acg ttg gtc aag ttt tat gct ccg tgg tgc   384
   Val Val Ile Gly Asp Leu Thr Leu Val Lys Phe Tyr Ala Pro Trp Cys
           35                  40                  45
   ggc cac tgc aag aca ctc gcc ccg gag ttt gta aag gcc gct gac atg   432
   Gly His Cys Lys Thr Leu Ala Pro Glu Phe Val Lys Ala Ala Asp Met
       50                  55                  60
   ctg gcc ggc atc gcg acc ctt gca gag gtc gat tgc acc aaa gaa gag   480
   Leu Ala Gly Ile Ala Thr Leu Ala Glu Val Asp Cys Thr Lys Glu Glu
    65                  70                  75                  80
   agc ctt gct gag aag tac gaa atc aag ggg ttc ccc acg ctg tac atc    528
   Ser Leu Ala Glu Lys Tyr Glu Ile Lys Gly Phe Pro Thr Leu Tyr Ile
                    85                  90                  95
   ttc cgt aac ggt gag aaa gtg aag atc tac gat ggt ccc cgc act gcc    576
   Phe Arg Asn Gly Glu Lys Val Lys Ile Tyr Asp Gly Pro Arg Thr Ala
               100                 105                 110
   gcc ggc atc gcg tcg tac atg aag gcg cat gtc ggt cca tcg atg aag    624
   Ala Gly Ile Ala Ser Tyr Met Lys Ala His Val Gly Pro Ser Met Lys
           115                 120                 125
   gcc atc tca acg gct gaa gag ctg gag gag ctc aag aag gag act ttc    672
   Ala Ile Ser Thr Ala Glu Glu Leu Glu Glu Leu Lys Lys Glu Thr Phe
       130                 135                 140
   ccg gtg tgc gtg gtg aag aca gcg agc acc gac tcg gag atg gcg tcg    720
   Pro Val Cys Val Val Lys Thr Ala Ser Thr Asp Ser Glu Met Ala Ser
   145                 150                 155                 160
   atg ata acc aag gtg gcg gac tct ctc cgc tcg cag atg aac ttt gtg    768
   Met Ile Thr Lys Val Ala Asp Ser Leu Arg Ser Gln Met Asn Phe Val
                   165                 170                 175
   ctc gtg acg gat gcg gcc atc tct ccg aat gat gcc atg gag tcg gtt    816
   Leu Val Thr Asp Ala Ala Ile Ser Pro Asn Asp Ala Met Glu Ser Val
               180                 185                 190
   acg gtg tat cgc aag aat gcg gag cgc gag gcg tac acc ggc gct aca    864
   Thr Val Tyr Arg Lys Asn Ala Glu Arg Glu Ala Tyr Thr Gly Ala Thr
           195                 200                 205
   cca atg acg gca gag tcg gtg aag agc ttt ctc acg agt gct gtg ttg    912
   Pro Met Thr Ala Glu Ser Val Lys Ser Phe Leu Thr Ser Ala Val Leu
       210                 215                 220
   gac tac ttt ggc gag ctc ggc cag gag agc ttt cag aag tac atg gaa    960
   Asp Tyr Phe Gly Glu Leu Gly Gln Glu Ser Phe Gln Lys Tyr Met Glu
   225                 230                 235                 240
   gcg aac aag gat aaa cct ctt ggg tgg gtg ttc atc gac aag aac acg    1008
   Ala Asn Lys Asp Lys Pro Leu Gly Trp Val Phe Ile Asp Lys Asn Thr
                   245                 250                 255
   gat tct gcg ttg aag ggg tca ctt gtg gcg gtg gcg gag aag tac cgc    1056
   Asp Ser Ala Leu Lys Gly Ser Leu Val Ala Val Ala Glu Lys Tyr Arg
               260                 265                 270
   tcg cag gtg ttg cta acc tac att gac ggc gat cag tac cgc ccc gtc    1104
   Ser Gln Val Leu Leu Thr Tyr Ile Asp Gly Asp Gln Tyr Arg Pro Val
           275                 280                 285
   tcg cgc cag ctg ggc att cct gag gat gcg aag ttc ccg gcg ttt gtg    1152
   Ser Arg Gln Leu Gly Ile Pro Glu Asp Ala Lys Phe Pro Ala Phe Val
       290                 295                 300
   gtc gat ttc gag cgc cgc cat cac gtg atg ggg acg gac acc cca gtc    1200
   Val Asp Phe Glu Arg Arg His His Val Met Gly Thr Asp Thr Pro Val
   305                 310                 315                 320
   acc tcc gag tct gtc gct gcg ttt gtg gag aag tat gtc aag ggc gag   1248
   Thr Ser Glu Ser Val Ala Ala Phe Val Glu Lys Tyr Val Lys Gly Glu
                   325                 330                 335
   acg aag cag acc gtg atg tcc gac gcg att ccc gct aag gag acg gtg   1296
   Thr Lys Gln Thr Val Met Ser Asp Ala Ile Pro Ala Lys Glu Thr Val
               340                 345                 350
   aac ggc ctc aca acg gtg gtg ggt cag act ttt gcg aag tac acg gac   1344
   Asn Gly Leu Thr Thr Val Val Gly Gln Thr Phe Ala Lys Tyr Thr Asp
           355                 360                 365
   ggc aca caa aac gtg atg ctg ctc ttc tac gcg ccg tgg tgc gga cac   1392
   Gly Thr Gln Asn Val Met Leu Leu Phe Tyr Ala Pro Trp Cys Gly His
       370                 375                 380
   tgc aag aag ctg cac ccc gtc tac gat aaa gta gcc aag agc ttc gag   1440
   Cys Lys Lys Leu His Pro Val Tyr Asp Lys Val Ala Lys Ser Phe Glu
   385                 390                 395                 400
   tct gag aat gtg atc att gcg aag atg gat gcc acg acg aac gac ttt   1488
   Ser Glu Asn Val Ile Ile Ala Lys Met Asp Ala Thr Thr Asn Asp Phe
                   405                 410                 415
   gac cgc gag aag ttt gag gtg tct gga ttt cca acg att tac ttc atc   1536
   Asp Arg Glu Lys Phe Glu Val Ser Gly Phe Pro Thr Ile Tyr Phe Ile
               420                 425                 430
   cca gcc ggc aag ccg cca atc gtg tac gag ggt ggc cgc acc gca gac   1584
   Pro Ala Gly Lys Pro Pro Ile Val Tyr Glu Gly Gly Arg Thr Ala Asp
           435                 440                 445
   gaa atc cag gtg ttt gtg aag tct cac ctg acc gcc tcc gcc gct cca   1632
   Glu Ile Gln Val Phe Val Lys Ser His Leu Thr Ala Ser Ala Ala Pro
       450                 455                 460
   tct ggc ggc cct tcc ggc aac agc gaa gag gaa gat ttg tag           1674
   Ser Gly Gly Pro Ser Gly Asn Ser Glu Glu Glu Asp Leu
   465                 470                 475
   gactgcaagg gatgtggcgt ttataggctg ccctgccttc ccttgctgtt tctatgacgg 1734
   attaggcttt tttttgttat atgtggggtg gtcaagagag tgccagggct ccttctttat 1794
   atccttgcgc tttcttttat tttgcttcct tgtgttgacg tctatgcatg cgtgctgtcg 1854
   acgactcttt gtcaacctgc gtcctatcta gtagcatcga tgtgaaaaga agagtagagg 1914
   gaggtaacga tgcgtgcgct ggctgccgtt ttcatgggcg caatttcgag aaggaaaatc 1974
   ggaaaatgga caggatagcg aaattagcgc aacgacaagg tcgtgcgtct ttctctatcg 2034
   gtcattaaat ttctgggctt tgtaacaatg aaagaagtca cacaaaaaaa aaaaaaaaaa 2094
<210> 2
   <211> 477
   <212> PRT
   <213> Leishmania major
<400> 2
   Met Gln Arg Ser Phe Leu Val Phe Val Leu Cys Ala Leu Leu Phe Cys
   1 5 10 15
   Val Ala Ser Ala Glu Val Gln Val Ala Thr Lys Asp Asn Phe Asp Lys
   20 25 30
   Val Val Ile Gly Asp Leu Thr Leu Val Lys Phe Tyr Ala Pro Trp Cys
   35 40 45
   Gly His Cys Lys Thr Leu Ala Pro Glu Phe Val Lys Ala Ala Asp Met
   50 55 60
   Leu Ala Gly Ile Ala Thr Leu Ala Glu Val Asp Cys Thr Lys Glu Glu
   65 70 75 80
   Ser Leu Ala Glu Lys Tyr Glu Ile Lys Gly Phe Pro Thr Leu Tyr Ile
   85 90 95
   Phe Arg Asn Gly Glu Lys Val Lys Ile Tyr Asp Gly Pro Arg Thr Ala
   100 105 110
   Ala Gly Ile Ala Ser Tyr Met Lys Ala His Val Gly Pro Ser Met Lys
   115 120 125
   Ala Ile Ser Thr Ala Glu Glu Leu Glu Glu Leu Lys Lys Glu Thr Phe
   130 135 140
   Pro Val Cys Val Val Lys Thr Ala Ser Thr Asp Ser Glu Met Ala Ser
   145 150 155 160
   Met Ile Thr Lys Val Ala Asp Ser Leu Arg Ser Gln Met Asn Phe Val
   165 170 175
   Leu Val Thr Asp Ala Ala Ile Ser Pro Asn Asp Ala Met Glu Ser Val
   180 185 190
   Thr Val Tyr Arg Lys Asn Ala Glu Arg Glu Ala Tyr Thr Gly Ala Thr
   195 200 205
   Pro Met Thr Ala Glu Ser Val Lys Ser Phe Leu Thr Ser Ala Val Leu
   210 215 220
   Asp Tyr Phe Gly Glu Leu Gly Gln Glu Ser Phe Gln Lys Tyr Met Glu
   225 230 235 240
   Ala Asn Lys Asp Lys Pro Leu Gly Trp Val Phe Ile Asp Lys Asn Thr
   245 250 255
   Asp Ser Ala Leu Lys Gly Ser Leu Val Ala Val Ala Glu Lys Tyr Arg
   260 265 270
   Ser Gln Val Leu Leu Thr Tyr Ile Asp Gly Asp Gln Tyr Arg Pro Val
   275 280 285
   Ser Arg Gln Leu Gly Ile Pro Glu Asp Ala Lys Phe Pro Ala Phe Val
   290 295 300
   Val Asp Phe Glu Arg Arg His His Val Met Gly Thr Asp Thr Pro Val
   305 310 315 320
   Thr Ser Glu Ser Val Ala Ala Phe Val Glu Lys Tyr Val Lys Gly Glu
   325 330 335
   Thr Lys Gln Thr Val Met Ser Asp Ala Ile Pro Ala Lys Glu Thr Val
   340 345 350
   Asn Gly Leu Thr Thr Val Val Gly Gln Thr Phe Ala Lys Tyr Thr Asp
   355 360 365
   Gly Thr Gln Asn Val Met Leu Leu Phe Tyr Ala Pro Trp Cys Gly His
   370 375 380
   Cys Lys Lys Leu His Pro Val Tyr Asp Lys Val Ala Lys Ser Phe Glu
   385 390 395 400
   Ser Glu Asn Val Ile Ile Ala Lys Met Asp Ala Thr Thr Asn Asp Phe
   405 410 415
   Asp Arg Glu Lys Phe Glu Val Ser Gly Phe Pro Thr Ile Tyr Phe Ile
   420 425 430
   Pro Ala Gly Lys Pro Pro Ile Val Tyr Glu Gly Gly Arg Thr Ala Asp
   435 440 445
   Glu Ile Gln Val Phe Val Lys Ser His Leu Thr Ala Ser Ala Ala Pro
   450 455 460
   Ser Gly Gly Pro Ser Gly Asn Ser Glu Glu Glu Asp Leu
   465 470 475
<210> 3
   <211> 467
   <212> PRT
   <213> Séquence artificielle
<220>
   <223> Description de la séquence artificielle: protéine recombinante
<400> 3
   Met Ala Glu Val Gln Val Ala Thr Lys Asp Asn Phe Asp Lys Val Val
   1 5 10 15
   Ile Gly Asp Leu Thr Leu Val Lys Phe Tyr Ala Pro Trp Cys Gly His
   20 25 30
   Cys Lys Thr Leu Ala Pro Glu Phe Val Lys Ala Ala Asp Met Leu Ala
   35 40 45
   Gly Ile Ala Thr Leu Ala Glu Val Asp Cys Thr Lys Glu Glu Ser Leu
   50 55 60
   Ala Glu Lys Tyr Glu Ile Lys Gly Phe Pro Thr Leu Tyr Ile Phe Arg
   65 70 75 80
   Asn Gly Glu Lys Val Lys Ile Tyr Asp Gly Pro Arg Thr Ala Ala Gly
                   85                  90                  95
   Ile Ala Ser Tyr Met Lys Ala His Val Gly Pro Ser Met Lys Ala Ile
               100                 105                 110
   Ser Thr Ala Glu Glu Leu Glu Glu Leu Lys Lys Glu Thr Phe Pro Val
           115                 120                 125
   Cys Val Val Lys Thr Ala Ser Thr Asp Ser Glu Met Ala Ser Met Ile
       130                 135                 140
   Thr Lys Val Ala Asp Ser Leu Arg Ser Gln Met Asn Phe Val Leu Val
   145                 150                 155                 160
   Thr Asp Ala Ala Ile Ser Pro Asn Asp Ala Met Glu Ser Val Thr Val
                   165                 170                 175
   Tyr Arg Lys Asn Ala Glu Arg Glu Ala Tyr Thr Gly Ala Thr Pro Met
               180                 185                 190
   Thr Ala Glu Ser Val Lys Ser Phe Leu Thr Ser Ala Val Leu Asp Tyr
           195                 200                 205
   Phe Gly Glu Leu Gly Gln Glu Ser Phe Gln Lys Tyr Met Glu Ala Asn
       210                 215                 220
   Lys Asp Lys Pro Leu Gly Trp Val Phe Ile Asp Lys Asn Thr Asp Ser
   225                 230                 235                 240
   Ala Leu Lys Gly Ser Leu Val Ala Val Ala Glu Lys Tyr Arg Ser Gln
                   245                 250                 255
   Val Leu Leu Thr Tyr Ile Asp Gly Asp Gln Tyr Arg Pro Val Ser Arg
               260                 265                 270
   Gln Leu Gly Ile Pro Glu Asp Ala Lys Phe Pro Ala Phe Val Val Asp
           275                 280                 285
   Phe Glu Arg Arg His His Val Met Gly Thr Asp Thr Pro Val Thr Ser
       290                 295                 300
   Glu Ser Val Ala Ala Phe Val Glu Lys Tyr Val Lys Gly Glu Thr Lys
   305                 310                 315                 320
   Gln Thr Val Met Ser Asp Ala Ile Pro Ala Lys Glu Thr Val Asn Gly
                   325                 330                 335
   Leu Thr Thr Val Val Gly Gln Thr Phe Ala Lys Tyr Thr Asp Gly Thr
               340                 345                 350
   Gln Asn Val Met Leu Leu Phe Tyr Ala Pro Trp Cys Gly His Cys Lys
           355                 360                 365
   Lys Leu His Pro Val Tyr Asp Lys Val Ala Lys Ser Phe Glu Ser Glu
       370                 375                 380
   Asn Val Ile Ile Ala Lys Met Asp Ala Thr Thr Asn Asp Phe Asp Arg
   385                 390                 395                 400
   Glu Lys Phe Glu Val Ser Gly Phe Pro Thr Ile Tyr Phe Ile Pro Ala
   405 410 415
   Gly Lys Pro Pro Ile Val Tyr Glu Gly Gly Arg Thr Ala Asp Glu Ile
   420 425 430
   Gln Val Phe Val Lys Ser His Leu Thr Ala Ser Ala Ala Pro Ser Gly
   435 440 445
   Gly Pro Ser Gly Asn Ser Glu Glu Glu Asp Leu Leu Glu His His His
   450 455 460
   His His His
   465
<210> 4
   <211> 497
   <212> PRT
   <213> T. brucei
<400> 4
   Met Arg Ala Ile Phe Leu Val Ala Leu Ala Leu Ala Thr Met Arg Glu
   1 5 10 15
   Ser Thr Ala Glu Ser Leu Lys Leu Thr Lys Glu Asn Phe Asn Glu Thr
   20 25 30
   Ile Ala Lys Ser Glu Ile Phe Leu Val Lys Phe Tyr Val Asp Thr Cys
   35 40 45
   Gly Tyr Cys Gln Met Leu Ala Pro Glu Trp Glu Lys Ala Ala Asn Glu
   50 55 60
   Thr Ile Asp Asn Ala Leu Met Gly Glu Val Asp Cys His Ser Gln Pro
   65 70 75 80
   Glu Leu Ala Ala Asn Phe Ser Ile Arg Gly Tyr Pro Thr Ile Ile Leu
   85 90 95
   Phe Arg Asn Gly Lys Glu Ala Glu His Tyr Gly Gly Ala Arg Thr Lys
   100 105 110
   Asp Asp Ile Ile Lys Tyr Ile Lys Ala Asn Val Gly Pro Ala Val Thr
   115 120 125
   Pro Ala Ser Asn Ala Glu Glu Val Thr Arg Ala Lys Glu Glu His Asp
   130 135 140
   Val Val Cys Val Gly Leu Thr Ala Asn Asn Ser Thr Ser Leu Ser Thr
   145 150 155 160
   Thr Leu Ala Glu Ala Ala Gln Ser Phe Arg Val Ser Leu Lys Phe Phe
   165 170 175
   Glu Ala Glu Pro Lys Leu Phe Pro Asp Glu Lys Pro Glu Thr Ile Val
   180 185 190
   Val Tyr Arg Lys Gly Gly Glu Lys Glu Val Tyr Asp Gly Pro Met Glu
   195 200 205
   Val Glu Lys Leu Thr Glu Phe Leu Gln Ile Ser Arg Val Ala Phe Gly
   210 215 220
   Gly Glu Ile Thr Pro Glu Asn Tyr Gln Tyr Tyr Ser Val Ile Lys Arg
   225 230 235 240
   Pro Val Gly Trp Ala Met Val Lys Pro Asn Glu Thr Ala Ser Ile Glu
   245 250 255
   Leu Lys Glu Ser Leu Thr Glu Val Gly Lys Lys Met Arg Ser His Met
   260 265 270
   Val Val Leu Trp Val Asn Ile Ser Lys His Pro Val Trp Arg Asp Phe
   275 280 285
   Gly Val Pro Glu Asp Ala Lys Tyr Pro Ala Phe Leu Ala Ile His Trp
   290 295 300
   Gly Ala Asn Tyr Leu His Ser Thr Ala Glu Val Val Thr Arg Glu Ser
   305 310 315 320
   Leu Glu Lys Phe Ile Leu Glu Phe Ala Ala Gly Arg Val Glu Pro Thr
   325 330 335
   Ile Lys Ser Leu Pro Val Pro Glu Val Glu Thr Val Asp Gly Lys Thr
   340 345 350
   Thr Ile Val Ala Lys Thr Met Gln Lys His Leu Thr Ser Gly Lys Asp
   355 360 365
   Met Leu Ile Leu Phe Phe Ala Pro Trp Cys Gly His Cys Lys Asn Phe
   370 375 380
   Ala Pro Thr Phe Asp Lys Ile Ala Lys Glu Phe Asp Ala Thr Asp Leu
   385 390 395 400
   Ile Val Ala Glu Leu Asp Ala Thr Ala Asn Tyr Val Asn Ser Ser Thr
   405 410 415
   Phe Thr Val Thr Ala Phe Pro Thr Val Phe Phe Val Pro Asn Gly Gly
   420 425 430
   Lys Pro Val Val Phe Glu Gly Glu Arg Ser Phe Glu Asn Val Tyr Glu
   435 440 445
   Phe Val Arg Lys His Val Thr Thr Phe Lys Val Ser Glu Lys Pro Ala
   450 455 460
   Asn Val Thr Glu Glu Lys Lys Ser Glu Glu Glu Asn Lys Ser Ser Lys
   465 470 475 480
   Ser Asn Glu Ser Asn Asp Ser Asn Glu Ser Asn Val Asp Lys Gln Asp
   485 490 495
   Leu
<210> 5
   <211> 502
   <212> PRT
   <213> H. jecorina
<400> 5
   Met Gln Gln Lys Arg Leu Thr Ala Ala Leu Val Ala Ala Leu Ala Ala
   1               5                   10                  15
   Val Val Ser Ala Glu Ser Asp Val Lys Ser Leu Thr Lys Asp Thr Phe
               20                  25                  30
   Asn Asp Phe Ile Asn Ser Asn Asp Leu Val Leu Ala Glu Ser Phe Ala
           35                  40                  45
   Pro Trp Cys Gly His Cys Lys Ala Leu Ala Pro Glu Tyr Glu Glu Ala
       50                  55                  60
   Ala Thr Thr Leu Lys Asp Lys Ser Ile Lys Leu Ala Lys Val Asp Cys
   65                  70                  75                  80
   Val Glu Glu Ala Asp Leu Cys Lys Glu His Gly Val Glu Gly Tyr Pro
                   85                  90                  95
   Thr Leu Lys Val Phe Arg Gly Leu Asp Lys Val Ala Pro Tyr Thr Gly
               100                 105                 110
   Pro Arg Lys Ala Asp Gly Ile Thr Ser Tyr Met Val Lys Gln Ser Leu
           115                 120                 125
   Pro Ala Val Ser Ala Leu Thr Lys Asp Thr Leu Glu Asp Phe Lys Thr
       130                 135                 140
   Ala Asp Lys Val Val Leu Val Ala Tyr Ile Ala Ala Asp Asp Lys Ala
   145                 150                 155                 160
   Ser Asn Glu Thr Phe Thr Ala Leu Ala Asn Glu Leu Arg Asp Thr Tyr
                   165                 170                 175
   Leu Phe Gly Gly Val Asn Asp Ala Ala Val Ala Glu Ala Glu Gly Val
               180                 185                 190
   Lys Phe Pro Ser Ile Val Leu Tyr Lys Ser Phe Asp Glu Gly Lys Asn
           195                 200                 205
   Val Phe Ser Glu Lys Phe Asp Ala Glu Ala Ile Arg Asn Phe Ala Gln
       210                 215                 220
   Val Ala Ala Thr Pro Leu Val Gly Glu Val Gly Pro Glu Thr Tyr Ala
   225                 230                 235                 240
   Gly Tyr Met Ser Ala Gly Ile Pro Leu Ala Tyr Ile Phe Ala Glu Thr
                   245                 250                 255
   Ala Glu Glu Arg Glu Asn Leu Ala Lys Thr Leu Lys Pro Val Ala Glu
               260                 265                 270
   Lys Tyr Lys Gly Lys Ile Asn Phe Ala Thr Ile Asp Ala Lys Asn Phe
   275 280 285
   Gly Ser His Ala Gly Asn Ile Asn Leu Lys Thr Asp Lys Phe Pro Ala
   290 295 300
   Phe Ala Ile His Asp Ile Glu Lys Asn Leu Lys Phe Pro Phe Asp Gln
   305 310 315 320
   Ser Lys Glu Ile Thr Glu Lys Asp Ile Ala Ala Phe Val Asp Gly Phe
   325 330 335
   Ser Ser Gly Lys Ile Glu Ala Ser Ile Lys Ser Glu Pro Ile Pro Glu
   340 345 350
   Thr Gln Glu Gly Pro Val Thr Val Val Val Ala His Ser Tyr Lys Asp
   355 360 365
   Ile Val Leu Asp Asp Lys Lys Asp Val Leu Ile Glu Phe Tyr Thr Pro
   370 375 380
   Trp Cys Gly His Cys Lys Ala Leu Ala Pro Lys Tyr Asp Glu Leu Ala
   385 390 395 400
   Ser Leu Tyr Ala Lys Ser Asp Phe Lys Asp Lys Val Val Ile Ala Lys
   405 410 415
   Val Asp Ala Thr Ala Asn Asp Val Pro Asp Glu Ile Gln Gly Phe Pro
   420 425 430
   Thr Ile Lys Leu Tyr Pro Ala Gly Asp Lys Lys Asn Pro Val Thr Tyr
   435 440 445
   Ser Gly Ala Arg Thr Val Glu Asp Phe Ile Glu Phe Ile Lys Glu Asn
   450 455 460
   Gly Lys Tyr Lys Ala Gly Val Glu Ile Pro Ala Glu Pro Thr Glu Glu
   465 470 475 480
   Ala Glu Ala Ser Glu Ser Lys Ala Ser Glu Glu Ala Lys Ala Ser Glu
   485 490 495
   Glu Thr His Asp Glu Leu
   500
<210> 6
   <211> 488
   <212> PRT
   <213> C. elegans
<400> 6
   Met Ile Trp Val Gln Ala Ala Leu Val Ala Ser Phe Leu Ala Phe Ala
    1               5                  10                  15
   Ser Ala Gly Gly Ala Val Leu Glu Tyr Thr Asp Gly Asn Phe Asp Asp
               20                  25                  30
   Leu Ile Gln Thr His Asp Ile Ala Leu Val Lys Phe Tyr Ala Pro Trp
            35                  40                  45
   Cys Gly His Cys Lys Lys Ile Ala Pro Glu Tyr Glu Arg Ala Ala Pro
       50                  55                  60
   Lys Leu Ala Ser Asn Asp Pro Pro Val Ala Leu Val Lys Val Asp Cys
   65                  70                  75                  80
   Thr Thr Glu Lys Thr Val Cys Asp Lys Phe Gly Val Lys Gly Phe Pro
                   85                  90                  95
   Thr Leu Lys Ile Phe Arg Asn Gly Val Pro Ala Gln Asp Tyr Asp Gly
               100                 105                 110
   Pro Arg Asp Ala Asp Gly Ile Val Lys Phe Met Arg Gly Gln Ser Gly
           115                 120                 125
   Pro Ser Ser Lys Glu Leu Lys Thr Val Ala Glu Phe Glu Lys Phe Thr
       130                 135                 140
   Gly Gly Asp Glu Asn Val Val Ile Gly Phe Phe Glu Ser Glu Ser Lys
   145                 150                 155                 160
   Leu Lys Asp Ser Tyr Leu Lys Val Ala Asp Thr Glu Arg Asp Arg Phe
                   165                 170                 175
   Ser Phe Ala His Thr Ser Asn Lys Asp Ile Ile Lys Lys Ala Gly Tyr
               180                 185                 190
   Ser Asp Asp Val Val Val Phe Val Pro Lys Lys Leu His Asn Lys Phe
           195                 200                 205
   Asp Thr Asn Glu Phe Lys Tyr Asp Gly Asn Tyr Asp Thr Asp Lys Ile
       210                 215                 220
   Lys Asn Phe Leu Val His Glu Thr Val Gly Phe Ala Gly Ile Arg Thr
   225                 230                 235                 240
   Gln Gly Asn Leu Phe Gln Phe Glu Gln Lys Pro Ile Val Ile Val Tyr
                   245                 250                 255
   Tyr Asn Val Asp Tyr Val Lys Asp Pro Lys Gly Ser Asn Tyr Trp Arg
               260                 265                 270
   Asn Arg Val Leu Lys Val Ala Gln Asn Tyr Lys Arg Lys Val Gln Phe
           275                 280                 285
   Ala Val Ser Asn Lys Glu Glu Phe Ser Ser Glu Ile Glu Thr Asn Gly
       290                 295                 300
   Leu Gly Glu Arg Lys Asp Ser Asp Lys Pro Ile Val Ala Ile Leu Thr
   305                 310                 315                 320
   Asn Glu Gly Lys Tyr Pro Met Asp Gln Glu Phe Ser Val Asp Asn Leu
                   325                 330                 335
   Gln Gln Phe Val Asp Glu Val Leu Ala Gly Asn Ala Glu Pro Tyr Met
               340                 345                 350
   Lys Ser Glu Pro Ile Pro Asp Glu Gln Gly Asp Val Lys Val Ala Val
           355                 360                 365
   Gly Lys Asn Phe Lys Glu Leu Ile Met Asp Ala Asp Lys Asp Val Leu
       370                 375                 380
   Ile Glu Phe Tyr Ala Pro Trp Cys Gly His Cys Lys Ser Leu Ala Pro
   385                 390                 395                 400
   Lys Tyr Glu Glu Leu Ala Glu Lys Leu Asn Lys Glu Asp Val Ile Ile
                   405                 410                 415
   Ala Lys Met Asp Ala Thr Ala Asn Asp Val Pro Pro Met Phe Glu Val
               420                 425                 430
   Arg Gly Phe Pro Thr Leu Phe Trp Leu Pro Lys Asn Ala Lys Ser Asn
           435                 440                 445
   Pro Ile Pro Tyr Asn Gly Gly Arg Glu Val Lys Asp Phe Val Ser Phe
       450                 455                 460
   Ile Ser Lys His Ser Thr Asp Gly Leu Lys Gly Phe Ser Arg Asp Gly
   465                 470                 475                 480
   Lys Lys Lys Lys Lys Thr Glu Leu
                   485
<210> 7
   <211> 532
   <212> PRT
   <213> C. reinhard
<400> 7
   Met Asn Arg Trp Asn Leu Leu Ala Leu Thr Leu Gly Leu Leu Leu Val
    1               5                  10                  15
   Ala Ala Pro Phe Thr Lys His Gln Phe Ala His Ala Ser Asp Glu Tyr
               20                  25                  30
   Glu Asp Asp Glu Glu Asp Asp Ala Pro Ala Ala Pro Lys Asp Asp Asp
           35                  40                  45
   Val Asp Val Thr Val Val Thr Val Lys Asn Trp Asp Glu Thr Val Lys
       50                  55                  60
   Lys Ser Lys Phe Ala Leu Val Glu Phe Tyr Ala Pro Trp Cys Gly His
   65                  70                  75                  80
   Cys Lys Thr Leu Lys Pro Glu Tyr Ala Lys Ala Ala Thr Ala Leu Lys
                   85                  90                  95
   Ala Ala Ala Pro Asp Ala Leu Ile Ala Lys Val Asp Ala Thr Gln Glu
               100                 105                 110
   Glu Ser Leu Ala Gln Lys Phe Gly Val Gln Gly Tyr Pro Thr Leu Lys
           115                 120                 125
   Trp Phe Val Asp Gly Glu Leu Ala Ser Asp Tyr Asn Gly Pro Arg Asp
       130                 135                 140
   Ala Asp Gly Ile Val Gly Trp Val Lys Lys Lys Thr Gly Pro Pro Ala
   145                 150                 155                 160
   Val Thr Val Glu Asp Ala Asp Lys Leu Lys Ser Leu Glu Ala Asp Ala
                   165                 170                 175
   Glu Val Val Val Val Gly Tyr Phe Lys Ala Leu Glu Gly Glu Ile Tyr
               180                 185                 190
   Asp Thr Phe Lys Ser Tyr Ala Ala Lys Thr Glu Asp Val Val Phe Val
           195                 200                 205
   Gln Thr Thr Ser Ala Asp Val Ala Lys Ala Ala Gly Leu Asp Ala Val
       210                 215                 220
   Asp Thr Val Ser Val Val Lys Asn Phe Ala Gly Glu Asp Arg Ala Thr
   225                 230                 235                 240
   Ala Val Leu Ala Thr Asp Ile Asp Thr Asp Ser Leu Thr Ala Phe Val
                   245                 250                 255
   Lys Ser Glu Lys Met Pro Pro Thr Ile Glu Phe Asn Gln Lys Asn Ser
               260                 265                 270
   Asp Lys Ile Phe Asn Ser Gly Ile Asn Lys Gln Leu Ile Leu Trp Thr
           275                 280                 285
   Thr Ala Asp Asp Leu Lys Ala Asp Ala Glu Ile Met Thr Val Phe Arg
       290                 295                 300
   Glu Ala Ser Lys Lys Phe Lys Gly Gln Leu Val Phe Val Thr Val Asn
   305                 310                 315                 320
   Asn Glu Gly Asp Gly Ala Asp Pro Val Thr Asn Phe Phe Gly Leu Lys
                   325                 330                 335
   Gly Ala Thr Ser Pro Val Leu Leu Gly Phe Phe Met Glu Lys Asn Lys
               340                 345                 350
   Lys Phe Arg Met Glu Gly Glu Phe Thr Ala Asp Asn Val Ala Lys Phe
           355                 360                 365
   Ala Glu Ser Val Val Asp Gly Thr Ala Gln Ala Val Leu Lys Ser Glu
       370                 375                 380
   Ala Ile Pro Glu Asp Pro Tyr Glu Asp Gly Val Tyr Lys Ile Val Gly
   385                 390                 395                 400
   Lys Thr Val Glu Ser Val Val Leu Asp Glu Thr Lys Asp Val Leu Leu
                   405                 410                 415
   Glu Val Tyr Ala Pro Trp Cys Gly His Cys Lys Lys Leu Glu Pro Ile
               420                 425                 430
   Tyr Lys Lys Leu Ala Lys Arg Phe Lys Lys Val Asp Ser Val Ile Ile
           435                 440                 445
   Ala Lys Met Asp Gly Thr Glu Asn Glu His Pro Glu Ile Glu Val Lys
   450 455 460
   Gly Phe Pro Thr Ile Leu Phe Tyr Pro Ala Gly Ser Asp Arg Thr Pro
   465 470 475 480
   Ile Val Phe Glu Gly Gly Asp Arg Ser Leu Lys Ser Leu Thr Lys Phe
   485 490 495
   Ile Lys Thr Asn Ala Lys Ile Pro Tyr Glu Leu Pro Lys Lys Gly Ser
   500 505 510
   Asp Gly Asp Glu Gly Thr Ser Asp Asp Lys Asp Lys Pro Ala Ser Asp
   515 520 525
   Lys Asp Glu Leu
   530
<210> 8
   <211> 496
   <212> PRT
   <213> D. melano
<400> 8
   Met Lys Phe Leu Ile Cys Ala Leu Phe Leu Ala Ala Ser Tyr Val Ala
   1 5 10 15
   Ala Ser Ala Glu Ala Glu Val Lys Val Glu Glu Gly Val Leu Val Ala
   20 25 30
   Thr Val Asp Asn Phe Lys Gln Leu Ile Ala Asp Asn Glu Phe Val Leu
   35 40 45
   Val Glu Phe Tyr Ala Pro Trp Cys Gly His Cys Lys Ala Leu Ala Pro
   50 55 60
   Glu Tyr Ala Lys Ala Ala Gln Gln Leu Ala Glu Lys Glu Ser Pro Ile
   65 70 75 80
   Lys Leu Ala Lys Val Asp Ala Thr Val Glu Gly Glu Leu Ala Glu Gln
   85 90 95
   Tyr Ala Val Arg Gly Tyr Pro Thr Leu Lys Phe Phe Arg Ser Gly Ser
   100 105 110
   Pro Val Glu Tyr Ser Gly Gly Arg Gln Ala Ala Asp Ile Ile Ala Trp
   115 120 125
   Val Thr Lys Lys Thr Gly Pro Pro Ala Lys Asp Leu Thr Ser Val Ala
   130 135 140
   Asp Ala Glu Gln Phe Leu Lys Asp Asn Glu Ile Ala Ile Ile Gly Phe
   145 150 155 160
   Phe Lys Asp Leu Glu Ser Glu Glu Ala Lys Thr Phe Thr Lys Val Ala
   165 170 175
   Asn Ala Leu Asp Ser Phe Val Phe Gly Val Ser Ser Asn Ala Asp Val
   180 185 190
   Ile Ala Lys Tyr Glu Ala Lys Asp Asn Gly Val Val Leu Phe Lys Pro
   195 200 205
   Phe Asp Asp Lys Lys Ser Val Phe Glu Gly Glu Leu Asn Glu Glu Asn
   210 215 220
   Leu Lys Lys Phe Ala Gln Val Gln Ser Leu Pro Leu Ile Val Asp Phe
   225 230 235 240
   Asn His Glu Ser Ala Ser Lys Ile Phe Gly Gly Ser Ile Lys Ser His
   245 250 255
   Leu Leu Phe Phe Val Ser Arg Glu Gly Gly His Ile Glu Lys Tyr Val
   260 265 270
   Asp Pro Leu Lys Glu Ile Ala Lys Lys Tyr Arg Asp Asp Ile Leu Phe
   275 280 285
   Val Thr Ile Ser Ser Asp Glu Glu Asp His Thr Arg Ile Phe Glu Phe
   290 295 300
   Phe Gly Met Asn Lys Glu Glu Val Pro Thr Ile Arg Leu Ile Lys Leu
   305 310 315 320
   Glu Glu Asp Met Ala Lys Tyr Lys Pro Glu Ser Asp Asp Leu Ser Ala
   325 330 335
   Glu Thr Ile Glu Ala Phe Leu Lys Lys Phe Leu Asp Gly Lys Leu Lys
   340 345 350
   Gln His Leu Leu Ser Gln Glu Leu Pro Glu Asp Trp Asp Lys Asn Pro
   355 360 365
   Val Lys Val Leu Val Ser Ser Asn Phe Glu Ser Val Ala Leu Asp Lys
   370 375 380
   Ser Lys Ser Val Leu Val Glu Phe Tyr Ala Pro Trp Cys Gly His Cys
   385 390 395 400
   Lys Gln Leu Ala Pro Ile Tyr Asp Gln Leu Ala Glu Lys Tyr Lys Asp
   405 410 415
   Asn Glu Asp Ile Val Ile Ala Lys Met Asp Ser Thr Ala Asn Glu Leu
   420 425 430
   Glu Ser Ile Lys Ile Ser Ser Phe Pro Thr Ile Lys Tyr Phe Arg Lys
   435 440 445
   Glu Asp Asn Lys Val Ile Asp Phe Asn Leu Asp Arg Thr Leu Asp Asp
   450 455 460
   Phe Val Lys Phe Leu Asp Ala Asn Gly Glu Val Ala Asp Ser Glu Pro
   465 470 475 480
   Val Glu Glu Thr Glu Glu Glu Glu Glu Ala Pro Lys Lys Asp Glu Leu
   485 490 495
<210> 9
   <211> 481
   <212> PRT
   <213> C. parvum
<400> 9
   Met Ile Gly Ile Arg Ser Leu Val Ser Ala Ala Phe Leu Gly Phe Ser
   1               5                   10                  15
   Cys Leu Ser Lys Val Val Leu Gly Gly Asp Glu Ala His Phe Ile Ser
               20                  25                  30
   Glu His Ile Thr Ser Leu Thr Ser Ser Asn Phe Glu Asp Phe Ile Lys
           35                  40                  45
   Ser Lys Glu His Val Ile Val Thr Phe Phe Ala Pro Trp Cys Gly His
       50                  55                  60
   Cys Thr Ala Leu Glu Pro Glu Phe Lys Ala Thr Cys Ala Glu Ile Ser
   65                  70                  75                  80
   Lys Leu Ser Pro Pro Val His Cys Gly Ser Val Asp Ala Thr Glu Asn
                   85                  90                  95
   Met Glu Leu Ala Gln Gln Tyr Gly Val Ser Gly Tyr Pro Thr Ile Lys
               100                 105                 110
   Phe Phe Ser Gly Ile Asp Ser Val Gln Asn Tyr Ser Gly Ala Arg Ser
           115                 120                 125
   Lys Asp Ala Phe Ile Lys Tyr Ile Lys Lys Leu Thr Gly Pro Ala Val
       130                 135                 140
   Gln Val Ala Glu Ser Glu Glu Ala Ile Lys Thr Ile Phe Ala Ser Ser
   145                 150                 155                 160
   Ser Ser Ala Phe Val Gly Arg Phe Thr Ser Lys Asp Ser Ala Glu Tyr
                   165                 170                 175
   Ala Val Phe Glu Lys Val Ala Ser Gly His Arg Glu His Asn Tyr Ala
               180                 185                 190
   Phe Ile Ala Phe Phe Gln Glu Gly Glu Gln Lys Leu Glu Val Leu His
           195                 200                 205
   Lys Asp Glu Glu Pro Val Ser Leu Pro Met Pro Lys Thr Val Glu Glu
       210                 215                 220
   Leu Glu Ala Lys Ile Ser Ile Met Asn Val Pro Leu Phe Ser Ala Ile
   225                 230                 235                 240
   Ser Ala Glu Asn Tyr Ser Leu Tyr Met Ser Arg Glu Gly Tyr Thr Pro
                   245                 250                 255
   Gly Ser Val Val Leu Thr Arg Thr Ser Pro Ser Met Leu Gln Thr Leu
   260 265 270
   Glu Arg Leu Gln Leu Ile Thr Glu Lys Ser Met Pro Leu Phe Ser Leu
   275 280 285
   Asp Thr Glu Gln Phe Gly Ser His Ala Thr Gln His Leu Leu Ile Glu
   290 295 300
   Lys Phe Pro Gly Leu Val Ile Gln Ser Val Asn Val Pro Ser Ile Arg
   305 310 315 320
   Tyr Met Tyr Gly Pro Ala Lys Phe Asp Ser Val Glu Pro Leu Lys Glu
   325 330 335
   Phe Met Lys Gln Val Ser Glu Gly Lys His Glu Leu Ser Ile Lys Ser
   340 345 350
   Glu Pro Ile Pro Ala Glu Gln Ser Gly Pro Val Thr Val Val Val Gly
   355 360 365
   Lys Thr Phe Glu Glu Ile Val Phe Arg Ser Asp Lys Asp Val Leu Leu
   370 375 380
   Glu Ile Tyr Ala Gln Trp Cys Gly His Cys Lys Asn Leu Glu Pro Ile
   385 390 395 400
   Tyr Asn Gln Leu Gly Glu Glu Tyr Lys Asp Asn Asp Lys Val Val Ile
   405 410 415
   Ala Lys Ile Asn Gly Pro Gln Asn Asp Ile Pro Tyr Glu Gly Phe Ser
   420 425 430
   Pro Arg Ala Phe Pro Thr Ile Leu Phe Val Lys Ala Gly Thr Arg Thr
   435 440 445
   Pro Ile Pro Tyr Asp Gly Lys Arg Thr Val Glu Ala Phe Lys Glu Phe
   450 455 460
   Ile Ser Glu His Ser Ser Phe Pro Gln Glu Lys Glu Ser Arg Asp Glu
   465 470 475 480
   Leu
<210> 10
   <211> 508
   <212> PRT
   <213> Homo sapiens
<400> 10

## Description

L'invention se situe dans le domaine de la lutte contre les leishmanioses. Elle résulte de la mise en évidence, à partir d'isolats sauvages de *Leishmania major,* d'un gène codant pour une protéine, désignée LmPDI, présentant deux régions identiques à la séquence (Cys-Gly-His-Cys) du site actif potentiel des protéines disulfides isomérases (PDI). Cette protéine LmPDI est exprimée de façon prédominante dans les isolats les plus virulents du parasite. Elle constitue d'une part, une nouvelle cible thérapeutique pour élaborer des médicaments anti-leishmaniose et, d'autre part, un élément nouveau pouvant entrer dans la composition de préparations immunogènes et éventuellement vaccinantes, destinées à protéger un hôte, humain ou animal, contre *Leishmania.*

Les leishmanioses constituent un groupe hétérogène de pathologies qui affectent plusieurs millions d'individus et sont dues à l'infection de l'hôte par un parasite protozoaire du genre *Leishmania.* L'expression clinique de l'infection est caractérisée par un grand polymorphisme, incluant l'infection asymptomatique, les formes cutanées simples ou récidivantes, les formes cutanées diffuses ou anergiques, les formes cutanéo-mucosales et les formes viscérales, mortelles en l'absence de traitement spécifique. En général, et selon la distribution géographique de la maladie, chaque espèce ou sous-espèce de *Leishmania* est responsable d'une forme clinique particulière; cependant, cette règle n'est pas stricte. De plus, dans un même foyer, une même espèce parasitaire peut être responsable de formes cliniques de sévérité variable. Cette diversité dans l'expression clinique de l'infection est due, au moins en partie, à une diversité dans la virulence du parasite.

Durant son cycle, le parasite alterne entre deux stades : le stade promastigote flagellé, qui se trouve dans le tube digestif de l'insecte vecteur, et le stade amastigote dans le macrophage de l'hôte. Les drogues anti-leishmaniennes sont difficiles à utiliser, entre autre à cause de leur toxicité et des résistances de plus en plus fréquentes développées par le parasite (Lira, Sundar et al. 1999). Par ailleurs, les vaccins récemment développés et testés n'ont pas montré, pour le moment, l'efficacité attendue (Sharifi, FeKri et al. 1998; Khalil, El Hassan et al. 2000).

L'absence d'outils pour le contrôle des leishmanioses est expliquée en partie par la complexité des cycles de transmission du parasite et par l'insuffisance des connaissances actuelles sur la biologie du parasite. Durant les dix dernières années, plusieurs molécules ayant un rôle fondamental dans la biologie et l'infectivité du parasite ont été identifiées. Des modifications des glyco-conjugués de surface, particulièrement le lipophospho-glycane (LPG), sont associées à des modifications dans l'infectivité et la virulence du parasite *Leishmania (L.) major* et *L. donovani* (Beverley and Turco 1998; Desjardins and Descoteaux 1998; Sacks, Modi et al. 2000; Spath, Epstein et al. 2000), ce qui ne semble pas être le cas pour *L*. *mexicana* (Ilg 2000; Ilg, Demar et al. 2001). Des molécules impliquées dans la biosynthèse du LPG : phosphomannose isomérase (Garami and Ilg 2001), LPG1 (Sacks, Modi et al. 2000; Spath, Epstein et al. 2000), LPG2 (Descoteaux, Luo et al. 1995) et galactosyl transférase (De and Roy 1999) ont été aussi associées à la virulence de *Leishmania.* D'autre facteurs de virulence ont été récemment décrits. Ils comprennent la famille des cystéine protéinases (Mottram, Brooks et al. 1998), les *mitogen-activated protein* (MAP)-kinases (Wiese 1998), le gène A2 (Zhang and Matlasheviski 1997), la glycoprotéine de surface gp63 (Chakrabarty, Mukherjee et al. 1996), la *kinetoplastid membrane protein* (KMP)-11 (Mukhopadhyay, Sen et al. 1998), la superoxyde dismutase (Paramchuk, Ismail et al. 1997), la trypanothione reductase (Dumas, Ouellette et al. 1997), et certains membres des protéines du choc thermique (HSP) (Hubel, Krobitsch et al. 1997).

La caractérisation des facteurs de virulence peut avoir des implications fondamentales pour le développement de nouveaux médicaments ou vaccins contre ces affections. En effet, le ciblage préférentiel d'une protéine impliquée dans la virulence du parasite permet d'éviter l'apparition inutile de résistances chez des souches peu dangereuses, résistances qui peuvent ensuite être transmises à d'autres souches. De plus, une mutation de la protéine de virulence ciblée, entraînant une résistance au médicament, peut dans ce cas entraîner aussi une diminution, voire une perte de la virulence du parasite, et donc un certain effet thérapeutique.

Durant les dernières décennies, plusieurs approches ont été utilisées pour aborder l'étude des facteurs de virulence du parasite *Leishmania.* Ces approches sont fondées sur des études génétiques, comme la complémentation de parasites mutés (Ryan, Garraway et al. 1993; Descoteaux, Luo et al. 1995; Desjardins and Descoteaux 1997; Wiese 1998), l'utilisation de la technique de l'invalidation de gène (Titus, Gueiros-Filho et al. 1995; Mottram, Souza et al. 1996; Dumas, Ouellette et al. 1997; Hubel, Krobitsch et al. 1997; Mottram, Brooks et al. 1998), ou l'analyse de gènes des résistance aux médicaments sur des parasites manipulés au laboratoire (Cotrim, Garrity et al. 1999; Perez-Victoria, Perez-Victoria et al. 2001). Ces études ont permis l'identification de plusieurs gènes importants pour la biologie du parasite, qui sont actuellement en cours de validation comme cibles de nouvelles drogues (Selzer, Chen et al. 1997; McKerrow, Engel et al. 1999), ou pour le développement et l'utilisation de mutants atténués comme vaccins vivants (Titus, Gueiros-Filho et al. 1995; Streit, Recker et al. 2001). Il est important de signaler que la quasi-totalité des études effectuées jusqu'à présent sur la virulence du parasite *Leishmania* sont basées soit sur des clones de laboratoire qui ont perdu leur virulence après une culture prolongée, soit sur des parasites génétiquement manipulés par des expériences de mutagénèse, d'invalidation ou de surexpression de gènes. Il est donc possible que les conclusions relatives aux gènes de virulence identifiés dans ces conditions ne soient pas réellement pertinentes en ce qui concerne la pathogénicité naturelle du parasite dans les foyers de transmission.

Dans le but d'étudier les bases moléculaires de la virulence du parasite, en évitant le biais méthodologique lié à l'utilisation de souches de laboratoire, les inventeurs ont dans un premier temps isolé des souches sauvages de *Leishmania (L.) major* présentant des niveaux différents de virulence. *L*. *major* est l'agent de la leishmaniose cutanée zoonotique (LCZ), qui sévit chez l'homme de façon épidémique sur une aire très large qui s'étend sans discontinuité de la Mauritanie jusqu'en Mongolie. Les inventeurs ont identifié des isolats de *L. major,* obtenus de lésions humaines de LCZ, tous obtenus durant la même saison de transmission, et qui diffèrent dans leur pathogénicité dans le modèle expérimental de l'infection des souris sensibles BALB/c (exemple 1). Les différences dans le pouvoir pathogène expérimental corrèlent avec des différences de croissance *in vitro,* ce qui reflète des variations dans la biologie de ces isolats sauvages.

La technique de "*Differential Display*" (Liang and Pardee 1992; Liang, Bauer et al. 1995) a ensuite été utilisée pour identifier des gènes différentiellement exprimés entre des isolats complètement différents dans leur pouvoir pathogène expérimental chez la souris BALB/c (deux isolats très virulents et deux autres peu virulents). Cette technique permet l'étude de gènes qui s'expriment à des niveaux différents sans connaître, au préalable, leur séquence. Trois transcrits préférentiellement exprimés dans les deux isolats les plus virulents ont ainsi été identifiés. L'un de ces transcrits a été entièrement caractérisé grâce au criblage d'une banque d'ADNc de *L. major.* L'analyse de la séquence a montré une homologie avec la famille des protéines disulfide isomérases (PDI, Erp60 et Erp72) des eucaryotes. Cette nouvelle protéine a été désignée LmPDI pour les raisons suivantes : tout comme les autres membres de la famille des PDI, (i) LmPDI possède deux domaines actifs CGHC, (ii) la région N-terminale de cette protéine contient une séquence signal potentielle et, dans la région carboxy-terminale, un signal potentiel pour la rétention dans le réticulum endoplasmique (EEDL), (iii) elle peut s'organiser sous forme de structure oligomèrique, (iv) la protéine recombinante produite dans *E*. *coli* exprime une activité PDI *in vitro.* Par ailleurs, en dehors des régions conservées sus-mentionnées, il y a très peu de similitudes entre LmPDI et les autres PDI déjà décrites. En fait, la famille des PDI comprend plusieurs molécules très divergentes impliquées dans les processus de maturation des protéines sécrétées dans le reticulum endoplasmique (Noiva 1999; Frand, Cuozzo et al. 2000). Les PDI sont des protéines multi-fonctionnelles qui interviennent dans les mécanismes complexes de rétention, réparation, régulation de l'expression, elles assistent les changements de conformation pour ne permettre la sortie du réticulum endoplasmique que des protéines correctement repliées. En plus de leurs fonctions enzymatiques (réduction et isomérisation), d'autres fonctions ont été récemment attribuées aux PDI ; elles incluent les activités chaperonnes, la fixation aux peptides et l'adhésion cellulaire (Ferrari and Soling 1999). Il est intéressant de souligner que LmPDI est exprimée de façon prédominante dans les isolats les plus virulents (exemple 2). Au total, l'ensemble de ces résultats suggère que LmPDI joue un rôle important dans la virulence naturelle du parasite *Leishmania,* et peut donc constituer une nouvelle cible pour la chimiothérapie ou la vaccination.

De plus, des données récentes sur l'implication de l'équivalent bactérien des PDI (Martin 1995; Ostermeier, De Sutter et al. 1996) (appelé DsbA pour *disulfide bond*) suggèrent le rôle que cette protéine peut jouer dans la pathogénicité de différents microorganismes (Yu and Kroll 1999). L'inactivation du gène DsbA affecte de façon dramatique la survie et la virulence de *Shigella flexneri* (Yu 1998; Yu, Edwards-Jones et al. 2000). DsbA est par ailleurs impliquée dans la genèse de l'entérotoxine de *Vibrio cholerae* (Peek and Taylor 1992; Yu, Webb et al. 1992). DsbA est aussi importante pour la pathogénicité des espèces de *Esherichia coli* pathogènes : chez les espèces pathogènes pour le tractus urinaire, elle catalyse la formation des ponts disulfures d'une protéine chaperonne spécifique du pili (Zhang and Donnenberg 1996). Chez les espèces entéro-pathogènes, elle est aussi requise pour la stabilité et la formation des pili (Hultgren, Abraham et al. 1993; Wang, Bjes et al. 2000).

La présente demande constitue la première description suggérant un rôle important des PDI comme facteur de virulence d'un parasite protozoaire. La LmPDI pourrait exercer ses effets en assistant les changements de conformation et la stabilité d'autres facteurs essentiels pour la biologie et la pathogénie du parasite *Leishmania.* L'identification de tels facteurs serait extrêmement intéressante pour une meilleure compréhension de la biologie de ce parasite, et pour la mise au point de nouveaux traitements ou vaccins contre les leishmanioses.

L'invention porte donc en premier lieu sur une protéine impliquée dans la virulence de *Leishmania,* comportant au moins un site (Cys-Gly-His-Cys) identique au site actif potentiel d'une protéine de la famille des protéines disulfides isomérases (PDI). Cette protéine est de préférence une protéine codée par le parasite lui-même.

En particulier, l'invention porte sur la protéine LmPDI de *Leishmania major,* de séquence SEQ ID No: 2, et sur tout variant fonctionnel de la LmPDI, présentant au moins 40% d'identité, et de préférence au moins 80%, avec la LmPDI.

On définira ici un « variant fonctionnel de la LmPDI » comme une protéine capable de complémenter la LmPDI dans un test d'infectivité effectué avec une souche de *L*. *major* dans laquelle le gène de la LmPDI aurait été inactivé. On désigne ici, par test d'infectivité, tout test permettant d'évaluer des propriétés biologiques relatives à la croissance du parasite classiquement associées à la virulence. En particulier, on peut citer les trois types de tests suivants :
- Cinétique de croissance en milieu liquide de la forme promastigote du parasite , par exemple par une technique du type de celle décrite au point 2 de l'exemple 5 ;
- Capacité d'infection des macrophages de souris cultivés en vitro, par une technique telle que celle décrite à l'exemple 6 et dans l'article de Kebaïer et al, 2001 ;
- Capacité d'induire la leishmaniose murine expérimentale par infection de souris sensibles (BALB/c par exemple). La technique est détaillée par exemple au point 3 de l'exemple 5, et dans l'article de Kebaïer et al, 2001.

Les pourcentages d'identité avec la LmPDJ seront évalués en utilisant le logiciel CLUSTAL W version 1.8 (Thompson, J.D., Higgins, D.G. et Gibson, T.J.), ou le logiciel BOXSHADE version 3.21 (Hoffman, K. et Baron, M.), qui ont donné des pourcentages d'identité de la LmPDI avec des protéines de la famille des PDI de plusieurs espèces entre 27 et 36 % (exemple 2).

Un deuxième mode de réalisation de l'invention concerne un polypeptide recombinant comportant au moins un fragment de plus de 10 acides aminés d'une protéine telle que celles définies ci-dessus, le cas échéant fusionné à un autre fragment polypeptidique, ledit polypeptide recombinant étant susceptible de déclencher une réaction immunologique contre un épitope de la LmPDI, lorsqu'il est administré à un animal. L'invention porte également sur un polypeptide recombinant comportant au moins un fragment de plus de 10 acides aminés d'une protéine telle que celles définies ci-dessus, le cas échéant fusionné à un autre fragment polypeptidique, ledit polypeptide recombinant étant susceptible d'être reconnu par des anticorps dirigés contre la protéine LmPDI.

Dans tout ce qui précède et suit, le terme « polypeptide » doit être compris au sens large, c'est-à-dire incluant des séquences d'au moins 10 acides aminés (ou davantage lorsque cela est précisé), comprenant ou non des motifs glycosylés ou des glycolipides, et quelle que soit sa structure, primaire, secondaire ou tertiaire. Le fragment de la LmPDI présent dans les polypeptides recombinants de l'invention décrits ci-dessus peut présenter une taille supérieure à 15, 20, 30, 50 ou 100 acides aminés, et même davantage.

La LmPDI, recombinante ou purifiée à partir de cellules infectées, ainsi qu'un polypeptide de l'invention, peuvent être utilisés pour immuniser un hôte, humain ou animal, afin de le protéger de la leishmaniose ou de produire et récupérer des anticorps dirigés contre la LmPDI, comme cela est décrit à l'exemple 2.

Un polypeptide recombinant particulier selon l'invention est la protéine LmPDI-(His)₆ de séquence SEQ ID No :3, décrite à l'exemple 2.

Un autre exemple de polypeptide recombinant selon l'invention est une protéine de fusion entre un fragment de la LmPDI, comportant au moins un épitope de la LmPDI, et un polypeptide porteur favorisant la présentation de ce fragment au système immunitaire. Il peut s'agir, notamment, d'une fusion de tout ou partie de la LmPDI avec un fragment de la β-lactamase, d'une anatoxine tétanique ou diphtérique, ou de tout autre polypeptide d'un organisme pathogène, notamment d'origine parasitaire, bactérienne ou virale.

Un autre aspect de l'invention concerne une séquence d'acide nucléique codant pour une protéine ou un polypeptide tels que ceux décrits ci-dessus. Un séquence d'acide nucléique préférée comprend la séquence codante de la LmPDI, de séquence SEQ ID No : 1, ou un fragment de cette séquence de taille supérieure ou égale à 30 nucléotides, de préférence supérieure à 100 nucléotides, codant pour un polypeptide comportant au moins un épitope caractéristique de la LmPDI.

L'invention porte aussi sur un vecteur d'acide nucléique comprenant une séquence d'acide nucléique de l'invention. Par exemple, il peut s'agir d'un plasmide, d'un cosmide, d'un phage, ou d'un virus. De façon préférée, un vecteur de l'invention permettra l'expression, dans une cellule hôte, d'une protéine ou d'un polypeptide selon l'invention. En particulier, un vecteur de l'invention peut permettre l'expression, dans une bactérie ou une cellule eucaryote, de la LmPDI.

L'invention porte aussi sur une cellule en culture comprenant un vecteur tel que ceux décrits ci-dessus. Cette cellule peut être une bactérie, une levure, une cellule d'insecte, une cellule de mammifère, ou tout autre type de cellule. Elle peut être utilisée soit pour exprimer et éventuellement produire une protéine ou un polypeptide selon l'invention, soit pour produire un vecteur qui servira ensuite à exprimer une protéine ou un polypeptide selon l'invention dans un autre type cellulaire en culture, ou *in vivo.* A titre purement illustratif et non limitatif, on peut citer les cellules CHO, VERO, BHK21 et les cellules d'insectes, comme types cellulaires utilisables *in vitro* dans le cadre de la présente invention. De même, on peut citer le BCG et Salmonella typhimurium comme cellules utilisables *in vivo.* Enfin, il est important de noter que l'administration à un individu, d'un vecteur viral, par exemple le virus de la vaccine, ou d'ADN, codant pour un polypeptide ou une protéine tels que décrits ci-dessus, à des fins vaccinales, entre également dans le cadre de la présente invention.

Un cellule particulière de l'invention est la souche bactérienne LmPDI-XL₁, déposée à la Collection Nationale de Culture des Microorganismes (CNCM) le 31/01/2001 sous le numéro I-2621. Cette souche est dérivée d'une bactérie XL1-Blue MRF' strain de génotype Δ(mrcA)183 Δ(mcrCB-hsdSMR-mrr)173endA1 supE44 thi-1 recA1 gyrA96 relA1 lac[F' proAB lacl^{q}Z ΔM15 Tn10 (Tet^{r})], transformée par le plasmide pBK-CMV-LmPDI. Ce plasmide correspond au plasmide pBK-CMV commercialisé par Stratagene (La Jolla, CA), dans lequel a été rajouté l'ADNc de la LmPDI, entre les sites de restriction *EcoR* I et *Xho* I*.*

L'invention porte également sur une sonde d'acide nucléique qui s'hybride spécifiquement, en conditions stringentes, avec la séquence nucléique de SEQ ID No : 1, permettant ainsi de déterminer la présence ou l'absence du gène de virulence codant pour la LmPDI, dans un échantillon biologique.

On définira ici des « conditions d'hybridation stringentes » comme des conditions qui permettent l'hybridation spécifique de deux molécules d'ADN à environ 65°C, par exemple dans une solution de 6X SSC, 0.5% SDS, 5X solution Denhardt's et 100 µg/ml of d'ADN non spécifique dénaturé, ou n'importe quelle solution de force ionique équivalente, et après une étape de lavage réalisée à 65°C, par exemple dans une solution d'au plus 0.2X SSC et 0.1% SDS, ou n'importe quelle solution de force ionique équivalente. Toutefois, la stringence des conditions peut être adaptée par l'homme du métier, en fonction de la taille de la séquence à hybrider, son contenu en nucléotides GC, et de n'importe quel autre paramètre, par exemple suivant les protocoles qui sont décrits dans in Sambrook et al., 2001 (Molecular Cloning : A Laboratory Manual, 3rd Ed., laboratory press, Cold Spring Harbor, New York).

Dans la définition ci-dessus, et dans l'ensemble du présent texte, le terme « spécifique » est à comprendre au sens large du terme, habituellement donné dans les laboratoires. Ainsi, une molécule A reconnaît spécifiquement une molécule B si, dans un mélange complexe, la molécule A a une affinité pour la molécule B significativement supérieure à son affinité pour les autres molécules du mélange, de telle façon qu'il soit possible de détecter la molécule B par l'intermédiaire de la molécule A.

Les conditions de stringence utilisées ici sont celles qui permettent de détecter les PDI des différentes espèces leishmaniennes et non celles de l'hôte et d'autres microorganismes en présence d'une sonde radiomarquée synthétisée à partir du cDNA de la LmPDI.

Par exemple, une sonde de l'invention, qui s'hybride spécifiquement avec la séquence SEQ ID No :1 en conditions stringentes, est telle qu'un *Southern blot* effectué en utilisant cette sonde marquée, présente, lorsqu'il est effectué sur un échantillon d'ADN de cellules infectées par une souche de *L. major* exprimant la LmPDI, au moins une bande nettement distincte, et d'intensité supérieure aux autres bandes (non spécifiques), ladite bande n'apparaissant pas sur un *Southern blot* effectué dans les mêmes conditions, sur un échantillon d'ADN de cellules non infectées par une souche de *L. major.*

Un autre aspect de l'invention est une amorce nucléotidique permettant l'amplification spécifique d'au moins une partie de la séquence de SEQ ID No : 1, à partir de cellules infectées par *Leishmania,* permettant ainsi de déterminer la présence ou l'absence du gène de virulence codant la LmPDI, dans un échantillon biologique. L'amplification sera dite « spécifique » dès lors que la réaction d'amplification, réalisée à partir de cellules contrôle non infectées par *Leishmania,* ne conduit à l'amplification significative d'aucune séquence, alors que la même réaction, réalisée sur un échantillon contenant la séquence nucléotidique de SEQ ID No :1, conduit à l'amplification d'au moins un fragment de cette séquence.

Les sondes et amorces mentionnées ci-dessus peuvent le cas échéant être marquées, et/ou présentées dans des trousses de diagnostic, qui font également partie de l'invention. En effet, il peut être avantageux de déterminer la présence et, éventuellement, le niveau d'expression du gène de la LmPDI lors d'une infection par *Leishmania,* par exemple pour déterminer la charge parasitaire et/ou l'opportunité d'un traitement impliquant l'utilisation d'un inhibiteur de la LmPDI.

Dans un autre mode de réalisation, l'invention porte sur des anticorps purifiés, reconnaissant spécifiquement la LmPDI. Il peut s'agir d'anticorps monoclonaux ou polyclonaux, humains, humanisés, ou animaux. Ces anticorps peuvent être purifiés, par exemple, sur une colonne d'affinité LmPDI, en utilisant le protocole décrit dans la partie expérimentale. De tels anticorps spécifiques de la LmPDI peuvent trouver plusieurs applications.

Ils peuvent servir à détecter la présence de la LmPDI dans un échantillon biologique, par exemple pour diagnostiquer une leishmaniose et/ou déterminer l'opportunité d'utiliser un inhibiteur de la LmPDI pour traiter cette leishmaniose.

L'invention concerne donc aussi une méthode de diagnostic *in vitro* d'une infection par un parasite responsable dé la Leishmaniose. Une telle méthode peut être réalisée en utilisant un polypeptide ou une protéine de l'invention, ou un anticorps dirigé contre cette protéine, ou encore à l'aide de sondes telles que décrites ci-dessus.

Une méthode de diagnostic particulière de l'invention comprend les étapes suivantes :
- la mise en contact d'au moins un anticorps selon l'invention avec un prélèvement biologique d'un sujet partiellement infecté par un parasite responsable le la Leishmaniose, dans des conditions permettant la formation d'un complexe immun entre ledit anticorps et les protéines antigéniques contenues dans le prélèvement,
- la détection dudit complexe.

La détection du complexe peut se faire à l'aide de n'importe quel moyen connu de l'homme du métier (réaction enzymatique, transfert de fluorescence ou autre).

Les anticorps de l'invention peuvent être compris dans des trousses de diagnostic, au même titre que les sondes et les amorces mentionnées ci-dessus.

Des trousses de diagnostic pour la mise en oeuvre de la méthode ci-dessus font partie intégrante de la présente invention.

A titre d'exemple, une telle trousse peut comprendre
- au moins un anticorps selon l'invention,
- un milieu approprié à la formation de complexe immun entre les protéines antigéniques contenues dans le prélèvement analysé et ledit anticorps,
- des réactifs permettant la détection des complexes ainsi formés,
- le cas échéant, des échantillons témoins.

Alternativement, les anticorps de l'invention peuvent entrer dans la composition d'un médicament destiné à la prophylaxie, à l'atténuation, ou au traitement de certaines leishmanioses.

Dans un autre mode de réalisation de l'invention, l'invention porte sur une composition immunogène, comportant une protéine et/ou un polypeptide recombinant et/ou une séquence d'acide nucléique et/ou un vecteur et/ou une cellule de l'invention tels que décrits précédemment, ladite composition immunogène étant capable de stimuler *in vitro* la prolifération de cellules mononucléées provenant d'individus ayant été en contact avec un parasite *Leishmania.* Une composition immunogène préférée de l'invention est capable de stimuler *in vitro* la prolifération de cellules mononucléées provenant d'individus ayant été en contact avec *Leishmania major*.

Dans une réalisation préférée des compositions immunogènes de l'invention, ces compositions présentent une formulation pharmaceutiquement acceptable pour être administrée à une hôte humain ou animal.

Les inventeurs ont montré que la LmPDI était susceptible d'induire *in vitro* la production de cytokines par des cellules mononucléées provenant d'individus ayant été en contact avec *L. major,* et que le profil d'expression des cytokines correspondait à celui observé lors d'une réponse immunitaire de type Th1 (exemple 3). Une composition immunogène telle que celles décrites ci-dessus, capable d'induire une réponse immunitaire de type Th1 lorsqu'elle est administrée à un hôte, humain ou animal, constitue donc une réalisation particulièrement préférée de l'invention.

L'invention porte également sur une composition vaccinante comportant une protéine, et/ou un polypeptide recombinant, et/ou une séquence d'acide nucléique, et/ou un vecteur, et/ou une cellule de l'invention telle que décrite précédemment, ladite composition vaccinante étant destinée à protéger un hôte, humain ou animal, contre la leishmaniose. De préférence, les compositions vaccinantes de l'invention sont formulées de façon acceptable sur le plan pharmaceutique pour être administrées à un hôte humain ou animal.

Une telle composition vaccinante peut se présenter sous forme de liquide à injecter au patient, par voie sous-cutanée ou intra-musculaire, ou sous forme de vaccin oral, sous forme de pommade, ou encore sous forme de particules liées à une séquence nucléotidique de l'invention, par exemple par adsorption d'ADN à la surface des particules. Cette dernière forme permet l'administration du vaccin par « bombardement » (ou « *gene gun* »). Il est important de noter que les formulations de compositions vaccinantes mentionnées ici le sont uniquement à titre d'exemples, et ne sont en aucun cas restrictives.

Les compositions immunogènes et/ou vaccinantes de l'invention peuvent aussi comporter un ou plusieurs antigène(s) hétérologue(s) vis-à-vis de *Leshmania,* et/ou une ou plusieurs séquence(s) d'acide nucléique codant pour de tels antigènes. Les compositions de l'invention peuvent donc déclencher une réaction immunologique vis-à-vis de plusieurs pathogènes différents et, le cas échéant, constituer des polyvaccins.

Le processus de vaccination, ainsi que les doses d'agent actif, devront être adaptés au type de vaccin utilisé et au mammifère auquel il est administré.

Des procédés de vaccination contre *Leishmania,* consistant à administrer à un hôte, humain ou animal, une composition comportant une protéine, et/ou un polypeptide recombinant, et/ou une séquence d'acide nucléique, et/ou un vecteur, et/ou une cellule de l'invention tels que ceux décrits plus haut, entrent également dans le cadre de la présente invention.

La mise en valeur du rôle de la LmPDI dans la virulence de *Leishmania* permet aussi d'envisager des nouvelles stratégies de recherche de molécules actives pour inhiber la croissance du parasite. En effet, il a été montré, qu'une molécule inhibitrice de la PDI, par exemple telle que la bacitracine ou l'acide chloromercurilbenzenesulfonique (pCMBS) inhibe la croissance de Leishmania en milieu liquide (exemple 4). L'invention porté donc aussi sur un procédé de criblage de molécules susceptibles d'inhiber la croissance de *Leishmania major,* comportant une étape d'évaluation de la capacité desdites molécules à inhiber l'activité de la LmPDI. Les Protéines Disulfides Isomérases présentent en général plusieurs activités telles que, notamment, des activités d'oxydo-réduction, d'isomérase, et de chaperonne. Les procédés de criblage selon l'invention peuvent porter sur l'inhibition de n'importe laquelle des fonctions de la LmPDI.

Dans un procédé de criblage particulier de l'invention, l'étape d'évaluation de la capacité d'une molécule à inhiber l'activité de la LmPDI est effectuée dans un test de réactivation de la RNase A réduite et dénaturée, comportant les étapes suivantes :
- incubation de la RNase A réduite et dénaturée en présence de - LmPDI, dans des conditions permettant sa réactivation,
- incubation de la RNase A réduite et dénaturée dans des conditions identiques à celles permettant sa réactivation par la LmPDI, en ajoutant la molécule à tester,
- comparaison des résultats obtenus en absence et en présence de la molécule à tester, un défaut de réactivation de la RNase A en présence de la molécule testée révélant que cette molécule possède une activité inhibitrice de la LmPDI.

N'importe quel autre test d'activité des PDI peut être utilisé dans les procédés de criblage selon l'invention, en particulier n'importe quel test dérivé du protocole initial décrit par Lyles et Gilbert (1991).

Un procédé de criblage selon l'invention peut aussi comporter un test d'inhibition de la croissance de *Leishmania major* en milieu liquide et, le cas échéant, un test d'inhibition de la croissance de *Leishmania major* dans un modèle murin de leishmaniose expérimentale. Un exemple d'un tel procédé est détaillé dans la partie expérimentale, à l'exemple 5.

Les molécules actives criblées par le procédé tel que défini ci-dessus sont caractérisées par leur capacité à inhiber ou à moduler la croissance de *Leishmania major.*

Les résultats obtenus avec la bacitracine, et présentés dans l'exemple 4, montrent qu'un inhibiteur des PDI peut inhiber la croissance de *Leishmania.* L'utilisation d'un ou plusieurs inhibiteurs des Protéines Disulfides Isomérases (PDI), pour la préparation d'une composition pharmaceutique destinée à la prophylaxie, à l'atténuation, ou au traitement d'une infection par *Leishmania,* fait donc aussi partie intégrante de l'invention. Parmi les composés possédant une activité anti-PDI et pouvant être utilisés suivant l'invention, on peut citer des anticorps anti-PDI ou anti-LmPDI, la bacitracine, la bacitracine de zinc, l'acide 5,5'-dithiobis(2-nitrobenzoïque) (DTNB), l'acide p-chloromercuribenzenesulfonique (pCMBS), ou l'acide tocinoïque.

Les compositions préparées suivant les utilisations ci-dessus sont de préférence administrables à un hôte humain ou animal, par voie topique, orale ou parentérale.

Selon un de ses aspects particuliers, l'invention porte sur l'utilisation de la bacitracine ou de la bacitracine de zinc comme inhibiteur de la croissance d'un parasite responsable de la leishmaniose ou comme agent actif contre une infection à *Leishmania.*

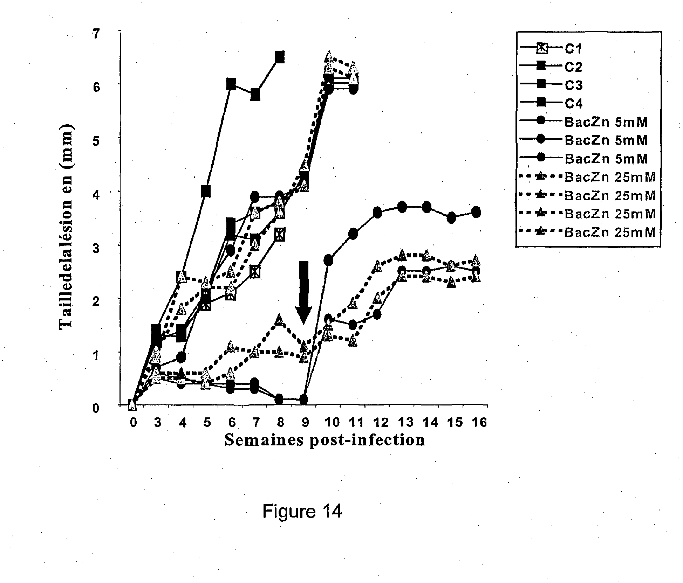

Bien entendu, une composition pharmaceutique destinée au traitement d'une infection par *Leishmania,* contenant un ou plusieurs inhibiteurs des Protéines Disulfides Isomérases (PDI), fait partie intégrante de l'invention. Une telle composition peut notamment contenir de la bacitracine ou de la bacitracine de zinc. La composition peut notamment être formulée pour une application topique, par exemple sous la forme d'une crème, d'un onguent, d'une pommade, ou d'un spray, sans que cela soit limitatif. Les inventeurs ont montré qu'une telle composition, appliquée localement sous forme de pommade au niveau du site d'injection du parasite chez des souris BALB/c, atténue la progression de la maladie (exemple 9 et figure 14).

La présente invention porte aussi sur une composition pharmaceutique destinée au traitement d'une infection par *Leishmania,* comportant au moins un anticorps spécifique de la LmPDI et/ou toute molécule qui inhibe l'activé PDI. Une telle composition est de préférence appropriée pour une administration topique, orale ou parentérale.

Des procédés de traitement des leishmanioses, comportant l'administration au patient, humain ou animal, d'un inhibiteur des PDI ou de la LmPDI, qu'il s'agisse d'un anticorps ou de tout autre type de molécule, entrent également dans le cadre de la présente invention.

Les exemples et figures ci-après, montrent les expériences biologiques qui ont été réalisées dans le cadre de cette invention et lui apportent le support expérimental requis, sans toutefois en limiter la portée. Ils illustrent aussi, de manière non restrictive, certains aspects de la mise en oeuvre et de l'intérêt de la présente invention.

Légende des figures :

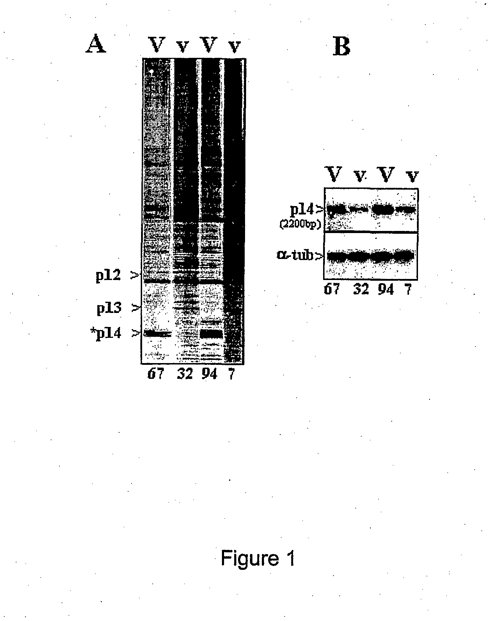

La figure 1 illustre l'analyse par "*Differential Display*" (DD) de l'expression de gènes de *Leishmania major* dans les deux isolats les plus virulents (94 et 67, V) et les deux isolats les moins virulents (32 et 07, v).
La figure 1A représente une portion d'un gel de séquençage après autoradiographie, montrant les produits amplifiés par PCR à l'aide d'un décamère arbitraire et d'une amorce oligo dT. Les ADNc différentiellement exprimés sont indiqués par des flèches. L'ADNc p14 est indiqué par un astérisque.
La figure 1B représente l'analyse par Northern blot de l'expression d'un gène identifié par la technique de DD entre les isolats les plus virulents (94 et 67, V) et les isolats les moins virulents (32 et 07, v). Les ARNm extraits à partir des promastigotes des différents isolats en phase stationnaire de croissance ont été hybridés avec la sonde radiomarquée p14. Après autoradiographie, les blots ont été déshybridés puis réhybridés avec une sonde spécifique du gène de l'α-tubuline de *L. major* (α -tub).

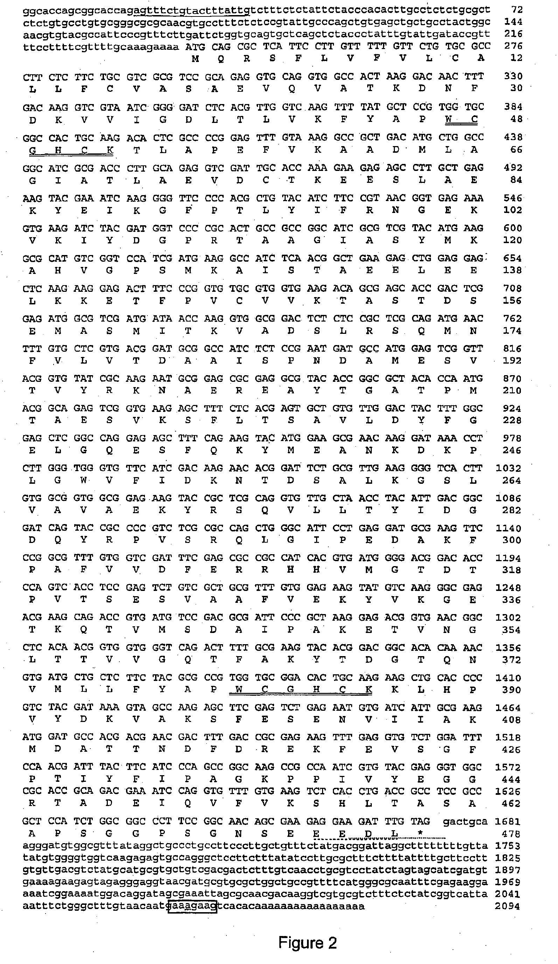

La figure 2 représente la séquence nucléotidique de l'ADNc (SEQ ID No : 1) de la LmPDI et sa séquence déduite en acides aminés (SEQ ID No : 2). Les nucléotides en minuscule représentent les régions non traduites. La séquence « leader » (SL) de 18 nt est soulignée et la séquence potentielle du signal de polyadénylation est encadrée. La séquence potentielle du peptide signal est représentée par des lettres en gras. Les sites actifs potentiels de la LmPDI sont doublement soulignés et la séquence probable de rétention dans le réticulum endoplasmique est indiquée d'un trait discontinu.

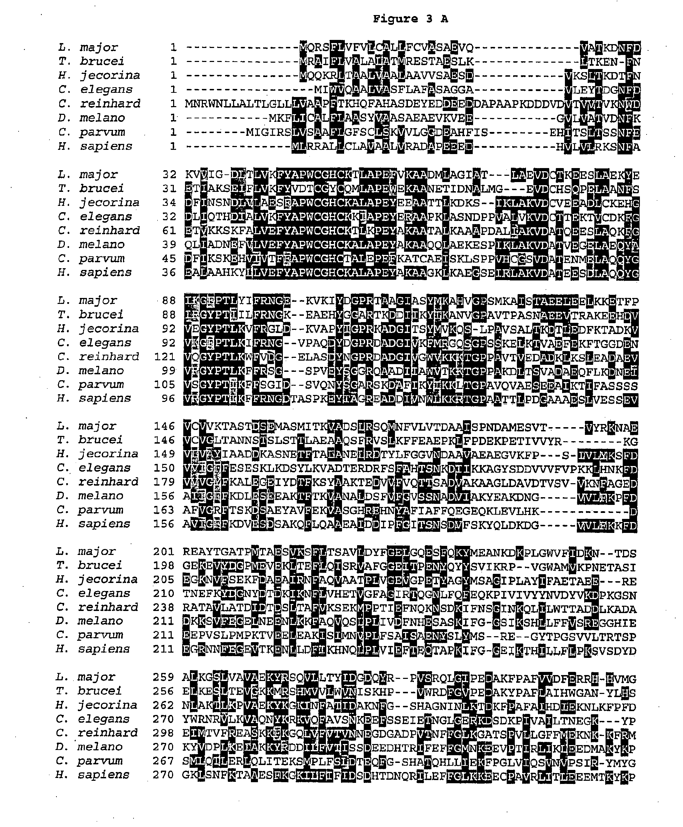

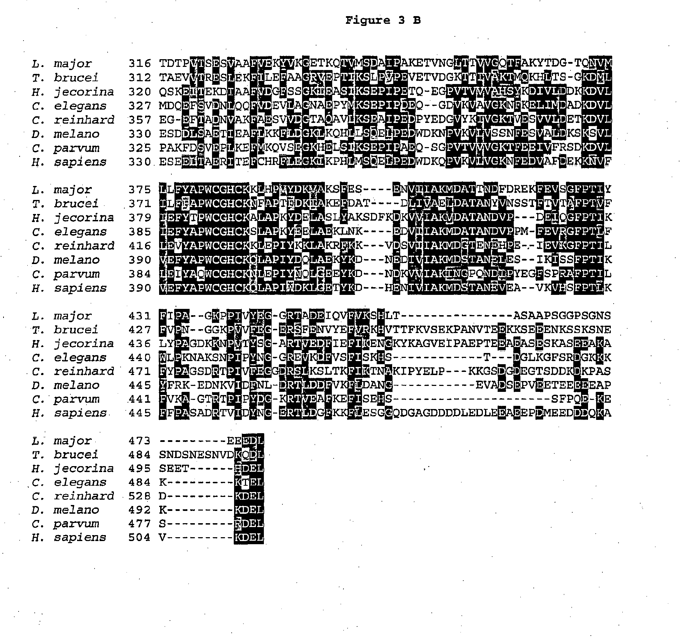

La figure 3 montre l'alignement de la séquence en acide aminés de la LmPDI avec des protéines disulfide isomérases de *Trypanosoma brucei* (*T. brucei*, GenBank accession no. : P12865), *Hypocrea jecorina (H. Jecorina,* 074568), *Caernorhabditis elegans* (*C. elegans,* 017908), Chlamydomonas reinhardtii (*C*. *reinhard,* 048949), *Drosophila melanogaster* (*D. melano,* P54399), *Cryptosporidium parvum* (*C*. *parvum*, Q27553), and *Homo sapiens* (*H*. *sapiens,* P072237). Les lettres encadrées en noir indiquent les acides aminés identiques et celles encadrées en gris indiquent les acides aminés similaires. Des «trous» ont été introduits pour obtenir le maximum de similarité entre les séquences alignées et sont indiqués par des tirets.
   Deux logiciels ont été utilisés pour la réalisation des alignements :
   - CLUSTAL W version 1.8
      Thompson, J.D., Higgins, D.G. and Gibson, T.J.
   - BOXSHADE version 3.21
      Hoffman, K. and Baron, M.

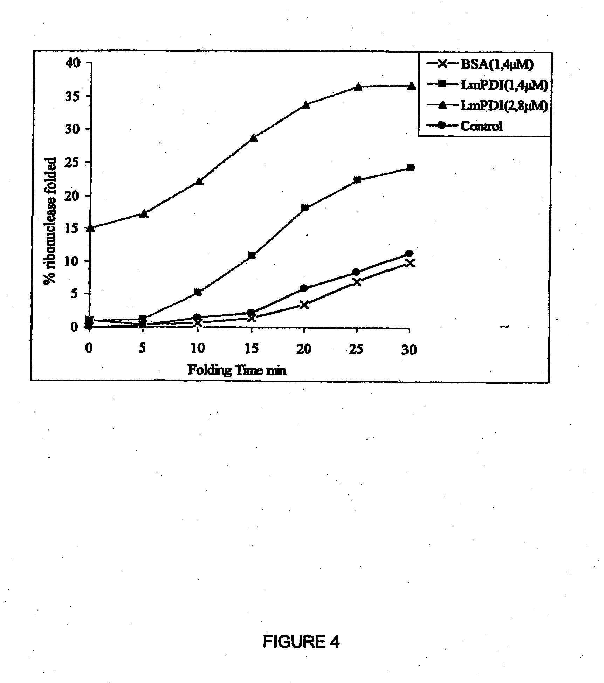

La figure 4 illustre l'analyse du rôle de la LmPDI recombinante dans la réactivation de la RNase réduite et dénaturée. La RNase A réduite et dénaturée (8µM) a été incubée dans un tampon contenant 4.5mM (cCMP), 1mM Glutathione GSH, 0.2mM Glutathione disulfide GSSH, 2mM EDTA et 100mM Tris-HCl pH 8 en présence de Sérum albumine bovine (BSA) (1.4µM) comme contrôle négatif, de Protéine Disulfide Isomérase bovine (1.4µM) comme contrôle positif, ou de LmPDI recombinante (1.4µM) pendant 30 minutes à 25°C. La réactivation de la RNase A est déterminée par la mesure de l'activité RNase A à 296nm toutes les 5 minutes pendant 30minutes (Lyles and Gilbert 1991).

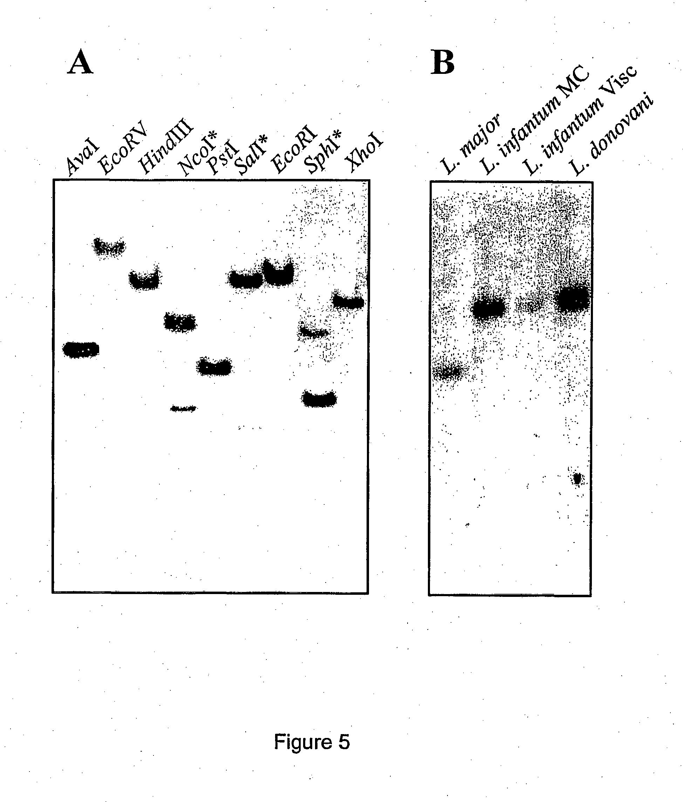

La figure 5A montre l'analyse par Southern blot du nombre de copies du gène de la LmPDI dans le génome de *Leishmania major.* 8µg de l'ADN génomique l'isolat 94 de *L. major* sont digérés par les enzymes de restriction suivantes: Aval, EcoRV, *Hind*III, *Pst*I, *EcoRI, Xho*I, *Nco*I, *Sac*I, *Sph*I. Les enzymes marquées d'un astérique coupent une seule fois à l'intérieur de l'ADNc de la LmPDI.
La figure 5B montre l'analyse par Southern blot du gène de la LmPDI dans différentes espèces de *Leishmania.* 8µg de l'ADN génomique de *L. major* (94), *L. infantum* dermotrope (*L*. *infantum* MC), *L. infantum* viscérotrope (*L. infantum* Visc) ; *L. donovani* sont digérés par l'enzyme *Pst* I. Les ADN génomiques sont hybridés dans ces expériences par la sonde représentant l'entière séquence de l'ADNc de la LmPDI.

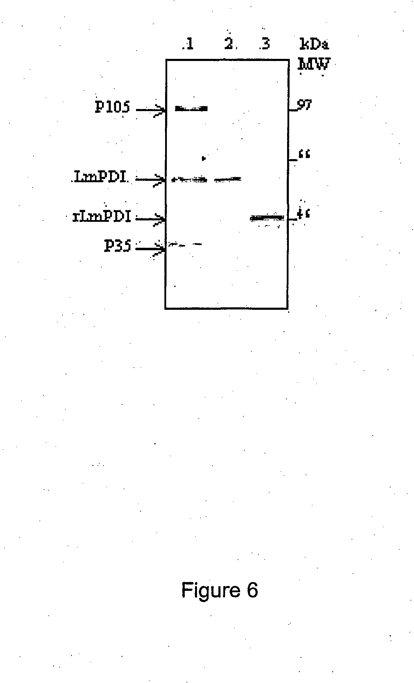

La figure 6 montre l'immunodétection de la LmPDI native chez *L. major* avec différentes préparations d'anticorps anti-LmPDI. 20µg de protéines totales de promastigotes de GLC 94 dans du tampon de Laemmli (piste 1) ou en présence de 0.5mM de DTT (piste 2) et 0.05µg de LmPDI produite dans les bactéries *E*. *coli* et purifiée (rLmPDI) (piste 3) sont soumis à une électrophorèse puis transférés sur une membrane de nitrocellulose, puis révélés avec l'immunsérum anti-LmPDI (piste 1) ou les anticorps anti-LmPDI purifiés sur colonne d'affinité (pistes 2 et 3).

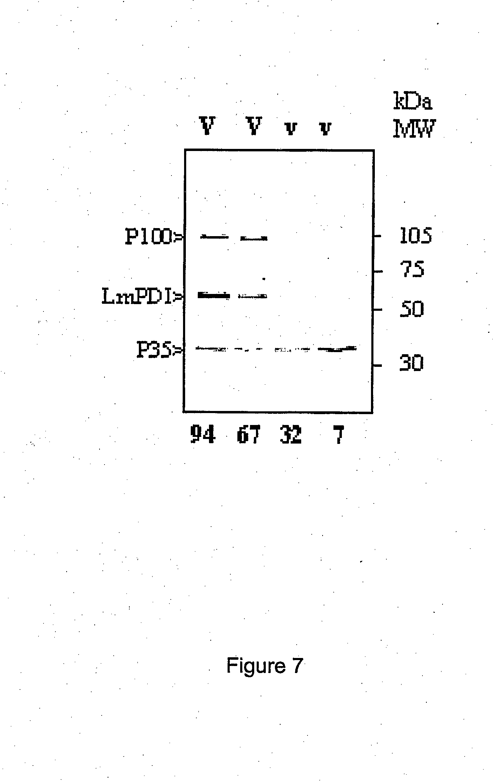

La figure 7 représente l'analyse par Western blot de l'expression de la LmPDI dans les deux isolats les plus virulents (94, 67, V) et les deux isolats les moins virulents (32, 7, v) de promastigotes. 20µg de protéines totales de promastigotes en phase stationnaire de croissance des différents isolats sont soumis à une électrophorèse puis transférés sur une membrane de nitrocellulose qui est incubée en présence de l'anticorps polyclonal anti-LmPDI. Les flèches (>) indiquent les 3 protéines reconnues par l'immunsérum anti-LmPDI.

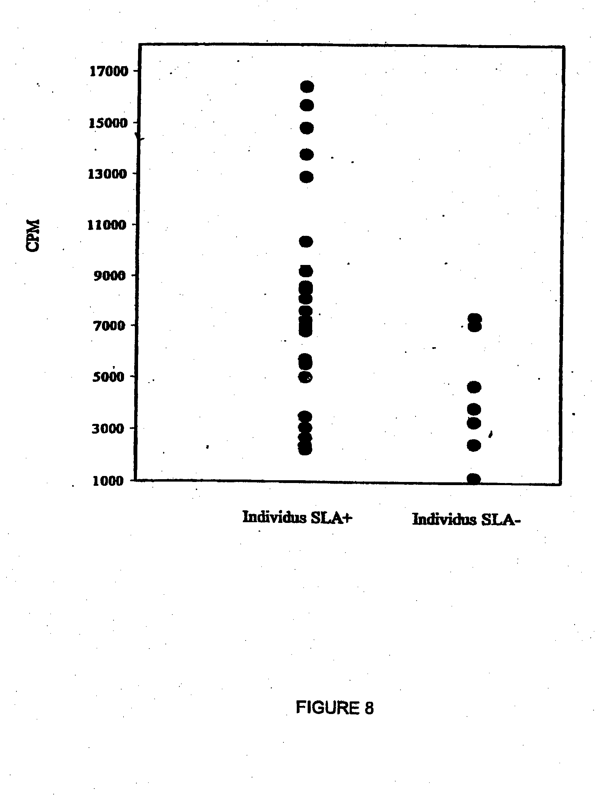

La figure 8 représente la prolifération des cellules mononucléées d'individus vivant dans un foyer de leishmaniose cutanée zoonotique en Tunisie, après incubation de LmPDI (5 µg/ml). Les cellules lympho-monocytaires sont récupérées, lavées par 3 centrifugations successives avec du milieu RPMI-PS/Glu (30 ml puis deux fois 10 ml) puis comptées et incubées à la concentration de 10⁶ cellules/ml de milieu en présence ou en absence d'une concentration de 5 µg/ml de LmPDI. Après 5 jours de culture, la stimulation lymphocytaire est estimée par incorporation de thymidine tritiée. Le résultat est exprimé en CPM.

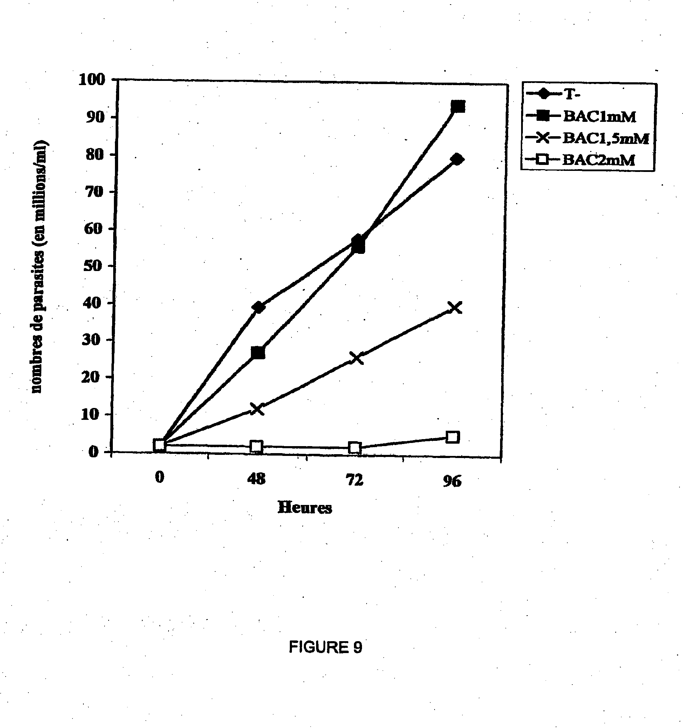

La figure 9 représente le résultat d'un test d'inhibition de la croissance des parasites (*L*. *major*) en milieu liquide, par la Bacitracine. Il s'agit des courbes de croissance sur 96 heures, de promastigotes de *L. major* en présence de 0, 1 mM, 1,5 mM, ou 2 mM de Bacitracine.

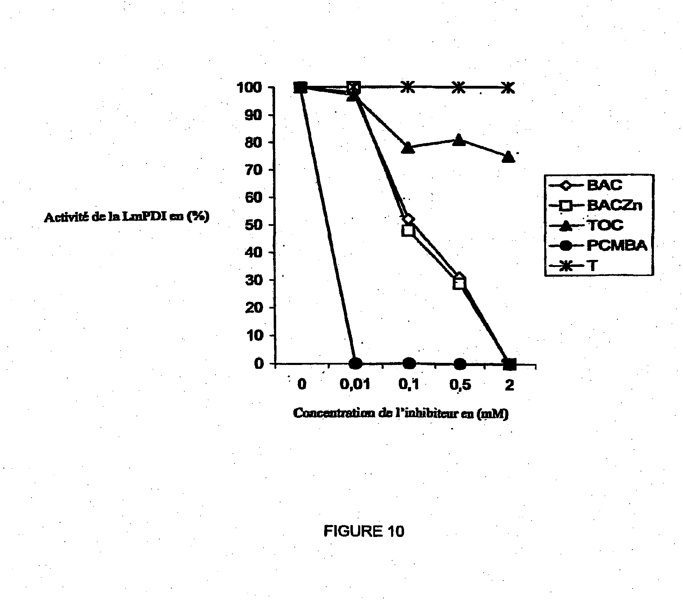

La figure 10 montre l'effet de la bacitracine (BAC), de la bacitracine de zinc (BACZn) du p-chloromercuribenzoic acid (pCMBA) et de l'acide tocinoic (TOC) sur l'activité *in-vitro* de la LmPDI recombinante. Différentes concentrations d'inhibiteurs (0 à 2mM) ont été utilisées pour suivre l'effet des inhibiteurs de PDI sur la capacité de la ImPDI à réactiver *in-vitro* la RNase A réduite et dénaturée. Comme contrôle positif (T) de ces expériences, la LmPDI sans inhibiteurs a été utilisée.

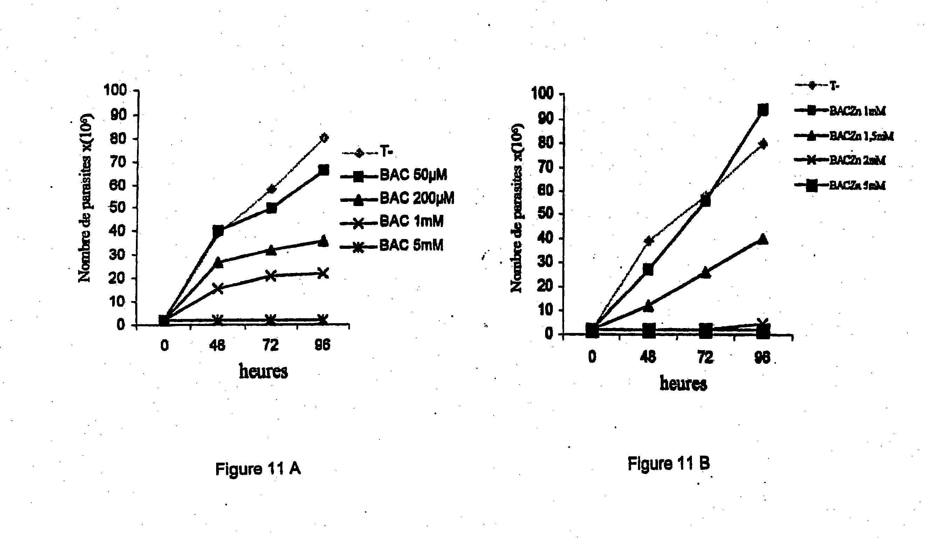

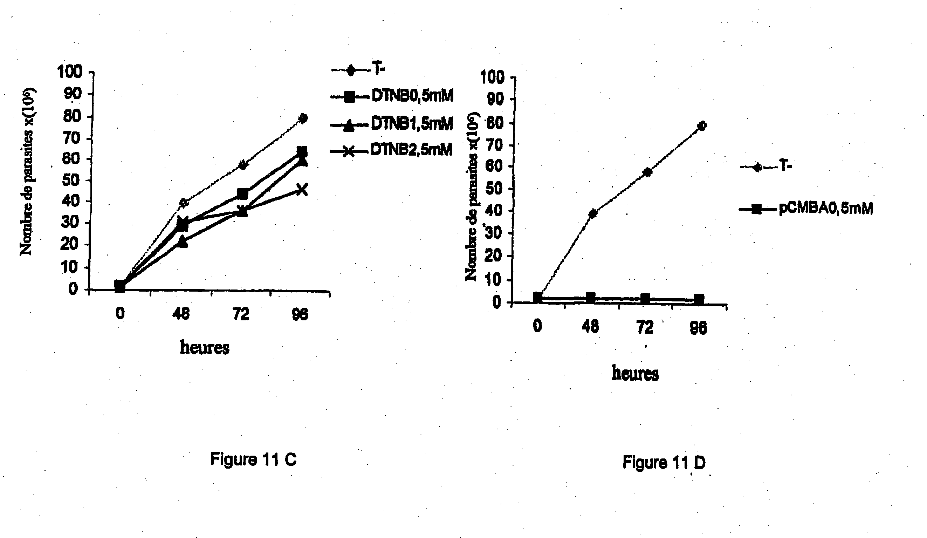

La figure 11 montre l'effet de la bacitracine (BAC) (figure 11A), de la bacitracine de zinc (BACZn) (figure 11B), du 5,5'-dithiobis(2-nitrobenzoic acid) (DTNMB) (figure 11C) et du p-chloromercuribenzoic acid (pCMBA) (figure 11 D) sur la croissance *in-vitro* des leishmanies en milieu liquide. Différentes concentrations d'inhibiteurs (0 à 5mM) ont été utlisées pour suivre l'effet des inhibiteurs de PDI sur la multiplication des parasites *in-vitro*. Comme contrôle de ces expériences, les parasites non traités avec les inhibiteurs (T-) ont été choisis.

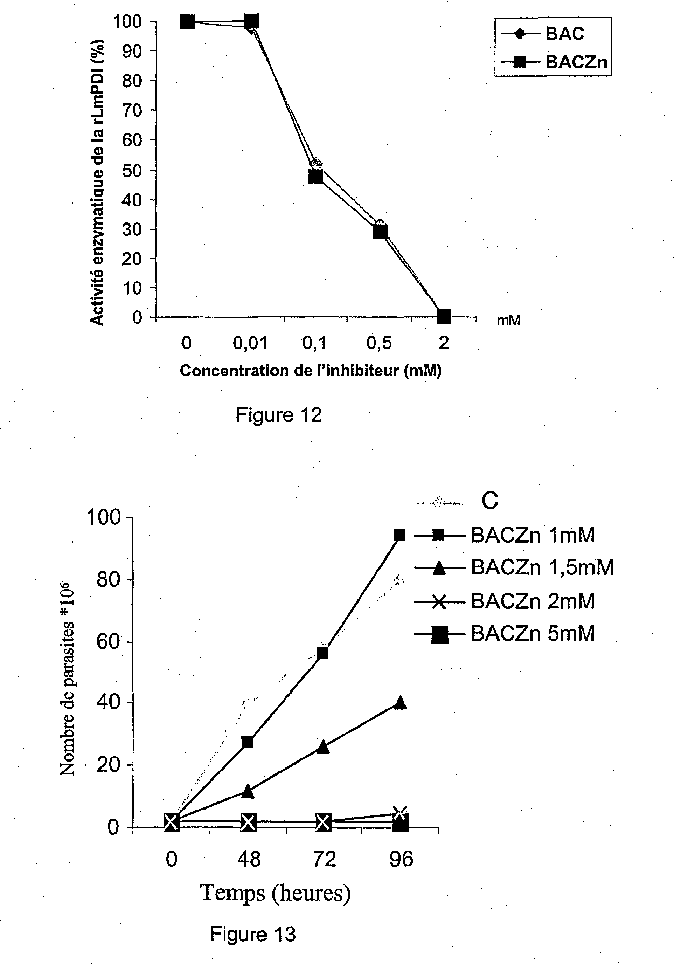

La figure 12 montre l'inhibition de l'activité de la rLmPDI par la bacitracine et la bacitracine de zinc. L'effet de la bacitracine (BAC) et de la bacitracine de zinc (BACZn) sur l'activité de la rLmPDI a été mesuré *in vitro.* Différentes concentrations d'inhibiteurs de BAC et de BACZn (0 à 2mM) ont été testées pour analyser leur effet sur la capacité de la rLmPDI à réactiver *in vitro* la RNase réduite et dénaturée. L'activité de la rLmPDI en l'absence d'inhibiteurs sert de contrôle positif.
La figure 13 illustre l'inhibition de la multiplication de promastigotes GLC94 par la bacitracine de zinc. L'effet de la bacitracine de zinc (BACZn) sur la multiplication de promastigotes GLC94 a été déterminé *in vitro.* Différentes concentrations d'inhibiteur ont été testées pour analyser leur effet sur la capacité des parasites à se multiplier *in vitro.* Des parasites cultivés dans du milieu complet en l'absence d'inhibiteurs représentent le contrôle (C).
La figure 14 montre l'effet de la bacitracine de zinc sur l'évolution de la maladie chez des souris BALB/c sensibles infectées avec des promastigotes de l'isolat GLC94. Des souris BALB/c sensibles ont été infectées avec 10⁶ promastigotes de l'isolat GLC94 dans le coussinet plantaire et traitées ou non avec de la bacitracine. Le traitement a été arrêté 9 semaines après infection (La flèche indique l'arrêt du traitement). Chaque courbe représente l'évolution de la taille d'une seule souris.

### Exemples :

L'ensemble des résultats expérimentaux présentés dans les exemples suivants ont été obtenus avec les matériels et méthodes suivants :

### Parasites et conditions de culture

Les isolats de *L. major* utilisés dans ce travail proviennent de lésions humaines de LCZ obtenus lors d'une étude résumée dans l'exemple 1. Les parasites ont été cultivés sur milieu NNN (milieu solide préparé à base d'agarose et de sang de lapin) à 26°C, et progressivement transférés dans du RPMI (SIGMA, St Louis, MO) contenant 2mM de L-Glutamine, 100U/ml de pénicilline, 100µg/ml de streptomycine et 10% de Sérum de Veau Foetal inactivé (milieu complet). Les promastigotes en phase logarithmique de croissance sont ajustés à 10⁶ parasites/ml dans un volume constant de milieu complet et incubés à 26°C. La phase stationnaire de croissance est atteinte après 4 à 6 jours avec des densités de parasites allant de 3.10⁷ à 8.10⁷. Ces promastigotes en phase stationnaire de croissance sont utilisés pour les extractions d'ARN et de protéines.

### Extraction d'ARN et « Differential Display »

Les ARN totaux sont extraits à l'aide du réactif « TRIZOL » (Gibco-BRL). Les ARN polyA⁺ sont purifiés par passage sur colonne d'oligo dT / cellulose à l'aide du kit « poly A⁺ ARN isolation kit » (Amersham-Pharmacia) selon les instructions du fabricant. 200ng d'ARNm ont été utilisés dans une réaction de reverse-transcription de 20µl contenant 1µM d'une amorce Oligo(dT)₁₁MN, avec M= A ou C ou G et N= A ou C ou G ou T (Genset), 1X Tampon First Strand (Gibco-BRL), 5µM dNTP (Amersham-Pharmacia), 10U de RNAsin (Promega) et 200U de Reverse-Transcriptase (Gibco-BRL).

Après incubation à 37°C pendant 1 heure, la réaction est stoppée par une incubation de 5 minutes à 95°C. Les ADNc sont amplifiés par PCR à l'aide d'une combinaison de 12 oligo dT et de 10 décamères arbitraires. La PCR est effectuée dans un volume de 20µL contenant 2µl de la réaction de reverse-transcription, 0.2µM de l'amorce 5', 1µM de l'amorce 3', 2µM de dNTP, 10µCi [α³⁵S]dATP, 1X tampon « Taq DNA polymerase reaction buffer » et 1 U de Taq polymérase (Amersham-Pharmacia). Les réactions sont incubées dans un thermocycleur Perkin-Elmer 9600 pendant 40 cycles à 94°C pendant 30s, 40°C pendant 60s et 72°C pendant 30s suivis d'un cycle à 72°C pendant 6 minutes. Les produits de PCR sont analysés sur un gel de séquençage à 6% d'acrylamide. Le gel est séché sous vide sur un papier Whatman 3MM et autoradiographié. Les ADNc différentiellement exprimés sont excisés du gel, élués et réamplifiés par PCR en présence des mêmes oligonucléotides et dans les conditions décrites précédemment. Les produits d'amplification sont clonés dans le vecteur pMOSblue à l'aide du kit « blunt-ended PCR cloning kit » (Amersham-Pharmacia) selon les instructions du fabricant. Les fragments clonés sont séquencés grâce au kit « Sequencing Ready Reaction Kit » (Perkin-Elmer) et analysés à l'aide du séquenceur automatique ABI 377.

### Analyse par Northern-Blot

200ng d'ARNm de promastigotes extraits durant la phase stationnaire de croissance des 4 isolats de *L. major* sont dénaturés, séparés sur un gel à 1,2% Agarose / 2,2 M formaldehyde et transférés par capillarité sur une membrane « Hybond N⁺ » (Amersham-Pharmacia). Les acides nucléiques sont ensuite fixés par une cuisson de 2 heures à 80°C. Les fragments d'ADNc différentiellement exprimés et de l'α-tubuline sont marqués avec de l'[α³² P]dCTP en utilisant le kit « megaprime DNA labelling system kit » (Amersham-Pharmacia). Les hybridations sont effectuées dans une solution à 1X Denhardt's/6X SSC/ 0,1% SDS/ 0,1mg.ml⁻¹ d'ADN de sperme de saumon toute la nuit à 65°C. Les membranes sont lavées à 65°C dans une solution contenant 0,1X SSC/ 0,1% SDS et autoradiographiées.

### Construction d'une banque d'ADNc et caractérisation de l'ADNc de la LmPDI

Une banque d'ADNc a été construite à partir de 5 µg d'ARNm de promastigotes de la souche la plus virulente (GLC94) dans le vecteur ZAPII, selon les instructions du fabricant (Stratagene). 6.10⁶ plages de lyse ont été criblées à l'aide de la sonde p14 marquée au [α³² P]P]dCTP en utilisant le kit « megaprime DNA labelling system kit » (Amersham-Pharmacia). Les plages de lyse d'intérêt sont prélevées et criblées de nouveau pour isoler les clones positifs des clones contaminants. Les clones positifs sont ensuite séquencés.

### Analyse par Southern-Blot

10µg d'ADN génomique extraits à partir de promastigotes de la souche la plus virulence GLC94 ont été digérés avec les enzymes de restriction indiquées dans la figure 5, et analysés sur un gel d'agarose 0,6%, puis transférés sur une membrane hybond N⁺ (Amersham-Pharmacia). La membrane est incubée en présence d'une sonde radioactive marquée au [α³² P] dCTP et correspondant au clone entier de l'ADNc de la LmPDI. Les membranes sont ensuite lavées dans une solution contenant 0,1X SSC/0,1 %SDS et autoradiographiées.

### Expression et purification de la protéine recombinante LmPDI dans les bactéries E. coli BL21

La séquence correspondant au cadre de lecture ouvert de l'ADNc de la LmPDI (1371pb) dépourvu de la séquence codant pour le peptide signal a été clonéé dans le vecteur d'expression bactérien pET-22b (Novagen). Les bactéries *E. coli* BL21 contenant le plasmide recombinant (pET-22b-LmPDI) sont cultivées dans du milieu LB puis la synthèse de la protéine recombinante est induite en présence de 1mM d'isopropyl-1-thio- -D-galactopyranoside (IPTG) pendant 4 heures. La protéine recombinante LmPDI-(His)₆ (SEQ ID No : 3) est purifiée par chromatographie d'affinité sur une colonne de nickel (Ni²⁺) (Amersham-Pharmacia). La pureté de la protéine produite est vérifiée par SDS-PAGE.

### Production d'un anticorps polyclonal anti-LmPDI et analyse de l'expression de la protéine native par immunoblot.

Un lapin a été immunisé par une injection intramusculaire de 500µg de la LmPDI recombinante purifiée émulsifiée (v/v) dans de l'adjuvant de Freund incomplet (IFA, Sigma). Le lapin a reçu deux injections additionnelles de 500µg de protéine recombinante, la première par voie intramusculaire 15 jours.après la première injection et la deuxième par voie intradermique 30 jours plus tard. Le lapin a été saigné 10 jours après la dernière injection, les sérums ont été récoltés et gardés à -80°C. Les lysats protéiques de promastigotes ont été dénaturés dans du tampon de Laemmli 1X pendant 10 minutes à 100°C, déposés sur un gel à 12% SDS-Acrylamide et électrotransférés sur une membrane de nitrocellulose (Millipore). Les membranes ont été incubées dans une solution de saturation PBS/0,1% Tween20 / 3% Lait écrémé à température ambiante pendant une heure puis dans la même solution contenant l'anticorps anti-LmPDI dilué au 1/1000^{ème} à 4°C pendant la nuit. Après 3 lavages dans du PBS/0,1% Tween20, les membranes ont été incubées en présence de l'anticorps secondaire anti-IgG de lapin couplé à la peroxydase (Amersham-Pharmacia, dilué au 1/1000) pendant une heure à température ambiante et lavées 3 fois dans du PBS/0,1% Tween20. Les complexes protéines-anticorps ont été révélés par la détection de l'activité peroxydase en utilisant le kit « ECL system » selon les instructions du fabricant (Amersham-Pharmacia).

### Préparation de la RNase A réduite et dénaturée

20mg de Ribonucléase (RNase A) purifiée ont été réduits et dénaturés à température ambiante pendant 18 heures dans un tampon contenant 0,15 M de DTT, 6 M de guanidine-HCl et 0,1 M de Tris-HCl à pH 8,6 avant d'être purifiés sur une colonne de sephadex G-25 équilibrée dans du 0,01 M HCl. La concentration des fractions de RNase A réduite et dénaturée a été déterminée à l'aide du coefficient d'extinction de 9200 M⁻¹cm⁻¹ à 275 nm. Les fractions sont conservées à -80°C pendant deux semaines.

### Réactivation de la RNase A en présence de la protéine recombinante LmPDI

La RNase A réduite et dénaturée (8 µM) a été incubée dans un tampon contenant 4,5 mM (cCMP), 1 mM Glutathione GSH, 0,2 mM Glutathione disulfide GSSH, 2 mM EDTA et 100 mM Tris-HCl pH 8 en présence de Sérum albumine bovine (BSA) (1,4 µM) comme contrôle négatif, de Protéine Disulfide Isomérase bovine (1,4 µM) comme contrôle positif, ou de LmPDI recombinante (1,4 µM) pendant 30 minutes à 25°C. La réactivation de la RNase A a été déterminée par la mesure de l'activité RNase A à 296nm toutes les 5 minutes comme précédemment décrit dans la littérature (Lyles and Gilbert 1991).

### Exemple 1 : sélection d'isolats de L. major, présentant différents niveaux de virulence

Les isolats de *L. major* utilisés dans ce travail proviennent de lésions humaines de LCZ obtenus lors d'une étude prospective menée en 1994-1995 à El Guettar, au sud de la Tunisie (Louzir, Melby et al. 1998). Ils ont été choisis parmi 19 isolats sur la base de leur pouvoir pathogène lors de l'infection expérimentale de souris sensibles BALB/c: 2.10⁶ amastigotes des diver isolats ont été injectés dans les coussinets des pattes arrières des souris BALB/c, et la progression de la lésion a été observée toutes les semaines pendant 9 semaines. Cinq semaines après l'infection, la production d'IL-4 et d'IFN-γ par des cellules mononucléées de ganglions lymphatiques activés *in vitro* par des antigènes du parasite a été mesurée.

Ces expériences ont montré d'une part, une grande hétérogénéité dans la progression de la maladie induite par les différents isolats de *L. major* et, d'autre part, que l'utilisation d'un même isolat conduit à des résultats reproductibles.

Les souches les plus virulentes ont induit les plus haut taux d'IL-4 et les plus bas taux d'IFN-y *in vitro,* 5 semaines après l'infection.

A partir de l'observation que l'expression clinique de l'infection par *L. major* est variable suivant les souches et reproductible avec chacune d'entre elles dans le modèle expérimental de l'infection des souris sensibles BALB/c, les inventeurs ont émis l'hypothèse que les gènes impliqués dans la virulence seraient différentiellement exprimés entre les isolats les plus virulents par rapport aux isolats les moins virulents. Une analyse préliminaire de l'expression d'un groupe de gènes préalablement décrits, par d'autres auteurs, et associés à la virulence du parasite, comprenant LPG1, LPG2, KMP-11, Cpc, Cpb, Hsp100, Gene B et gp63, a été réalisée par une technique de transcription inverse et d'amplification génique quantitative. Cette analyse n'a pas montré de différence entre les isolats de *L. major* exprimant une pathogénicité différente chez les souris BALB/c (Kebaier, Louzir et al. 2001).

Deux isolats, MHOM/TN/94/GLC94 et MHOM/TN/94/GLC67 (GLC94 et GLC67 respectivement) qui induisent des lésions graves, évoluant rapidement et représentant les isolats les plus virulents et 2 isolats MHOM/TN/94/GLC07 et MHOM/TN/94/GLC32 (GLC07 et GLC32 respectivement), induisant une maladie expérimentale moins sévère et représentant des isolats peu virulents, ont été sélectionnés pour poursuivre la recherche de gènes de virulence potentiellement exprimés à des niveaux différents suivant les souches.

### Exemple 2 : Identification par « Differential Display », d'une nouvelle protéine disulfide isomérase LmPDI de Leishmania major, impliquée dans la virulence naturelle du parasite

### 1. Identification des gènes différentiellement exprimés dans les isolats virulents et les isolats peu virulents de L. major

Les ARNm ont été tout d'abord purifiés à partir des promastigotes de deux isolats très virulents (GLC94 et GLC67) et de deux isolats peu virulents (GLC 32 et 07) puis reverse-transcrits en ADNc en utilisant des amorces Oligo(dT)₁₁MN, avec M= A ou C ou G et N= A ou C ou G ou T. L'amorce utilisée lors des expériences de differential display est :

Les réactions d'amplification ont été réalisées par PCR à l'aide des mêmes oligo-dT utilisés lors de la réaction de reverse transcription et combinés avec 10 amorces arbitraires, telles que décrites dans la littérature scientifique (Liang and Pardee 1992; Liang, Bauer et al. 1995; Heard, Lewis et al. 1996).

Au total, 60 combinaisons d'amorces ont été réalisées et analysées. L'analyse sur gel de polyacrylamide des produits d'amplification utilisant différentes combinaisons d'amorces a permis de montrer que les gènes des différents isolats de *L. major* (très virulents et peu virulents) expriment, dans approximativement 95% des cas, les mêmes ARNm et à des niveaux équivalents. Seuls, 25 messagers semblent être différentiellement exprimés entre les isolats très virulents et peu virulents (figure 1A). Les ADNc différentiellement exprimées ont été-tout-d'abord isolés du gel d'acrylamide puis réamplifiés à l'aide des mêmes combinaisons d'amorce utilisées lors de la première PCR et enfin, clonés dans le vecteur pMOS. Le séquençage des différents clones a permis de montrer qu'un certain nombre d'entre eux étaient identiques.

L'analyse de l'ARNm issu des différents isolats de *L. major* par Northern-blot en utilisant les 14 fragments differentillement exprimés comme sonde a permis de montrer que 3 clones parmi les 14 isolés présentent une expression différentielle entre les isolats très virulents et les isolats peu virulents. Un de ces clones, le clone p14, a été caractérisé. Par Northern blot, la sonde correspondant au clone p14 s'hybride spécifiquement avec un transcrit de taille approximative de 2,2kb, qui est préférentiellement exprimé dans les 2 isolats les plus virulents par rapport aux 2 isolats les moins virulents (figure 1B). Ceci confirme le résultat obtenu par la technique de *Differential Display*. Le clone p14 a été entièrement séquencé et la taille de ce clone est de 339pb. La comparaison de la séquence nucléotidique de ce fragment avec les séquences décrites dans les banques de données (GenBank et EMBL) n'a pas permis d'identifier de séquence signicativement homologue. Ceci peut être dû au fait que le clone p14 correspond à la région 3' terminale non traduite du messager.

### 2. Clonage et analyse de la séquence entière de l'ADNc p14

Pour isoler la séquence entière de l'ADNc correspondant au clone p14, le fragment de 339pb a été utilisé pour cribler une banque d'ADNc de promastigotes de l'isolat GLC94. Deux clones positifs ont été isolés à partir de 6X10⁵ clones recombinants analysés. La figure 2 montre la séquence nucléotidique du clone le plus long, qui est de 2094pb (SEQ ID No :1). Ce clone présente un cadre de lecture ouvert codant pour un polypeptide de 477 acides aminés (aa), avec un poids moléculaire théorique de 52,4 kDa et un point isoélectrique de 5,22. La région N-terminale de cette protéine correspond à un peptide signal potentiel d'export vers le réticulum endoplasmique, de 20 aa. La région 5' non traduite contient une séquence « *splice leader* » caractéristique des *Leishmania* et la région 3' non traduite contient une queue poly A précédée d'un site potentiel de polyadénylation (figure 2).

La séquence peptidique du clone isolé montre 27-36% d'identité avec les protéines de la famille des Protéine Disulfide Isomérases (PDI et Erp) de plusieurs espèces (figure 3). De plus, cette protéine contient deux régions aux résidus 47-52 et 381-386 qui sont identiques aux sites actifs potentiels (Cys-Gly-His-Cys, ou CGHC) des PDI, Erp et protéines de la famille des thioredoxines. La partie C-terminale montre un signal potentiel de rétention dans le réticulum endoplasmique de type KDEL (EEDL) aux résidus 474-477 suggérant que, comme les PDI et Erp, cette protéine se retrouve dans la lumière du réticulum endoplasmique. P14 est donc une protéine de la famille des Protéine Disulfide isomérases de *L. major.* Elle a été dénommée LmPDI (figure 2 et 3).

Pour déterminer si la LmPDI est dotée d'une activité thio-disulfide oxydoréductase comme cela a été démontré pour la plupart des protéines disulfide isomérases décrites, la capacité de la protéine recombinante LmPDI à renaturer la RNase A dénaturée a été étudiée. La protéine recombinante LmPDI a été synthétisée chez *E. coli* puis purifiée et utilisée dans un test, *in vitro,* de réactivation de la Rnase. Les résultats obtenus montrent que la LmPDI est capable de restaurer l'activité RNase A de manière similaire à celle de la PDI bovine, utilisée comme contrôle (figure 4)

Pour identifier le nombre de copies du gène codant pour le LmPDI, les inventeurs ont effectué une hybridation de type Southern blot en utilisant comme sonde le fragment d'ADNc de la LmPDI marquée au ³²P. Les résultats obtenus montrent généralement une bande unique, sauf pour les enzymes qui présentent un site de coupure au sein de l'ADNc de la LmPDI (figure 5A). Le gène codant pour la LmPDI est donc probablement présent en une copie unique dans le génome de *L. major.* De plus, le gène de la LmPDI apparaît conservé dans différentes espèces de *Leishmania* testées (*Leishmania infantum*, dermotrope, et un viscérotrope, *Leishmania donovani*) (figure 5B).

### 3. Analyse par immunoblot de l'expression de la LmPDI

Pour caractériser l'expression de la protéine native, un lapin a été immunisé avec la protéine recombinante LmPDI synthétisée chez *E. coli* et purifiée par chromatographie d'affinité. Par immunoblot, les inventeurs ont montré que l'anticorps polyclonal anti-LmPDI obtenu reconnaît fortement une protéine de la taille attendue (55kDa) dans les lysats de promastigotes en phase stationnaire de croissance de GLC94 (figure 6). D'autre part, deux autres protéines sont détectées. La première présente un poids moléculaire de 10 5kDa, ce qui correspond à environ deux fois celui de la LmPDI, et la deuxième a un poids moléculaire de 35 kDa. Afin de vérifier si la protéine de 105 kDa correspond à un dimère de la LmPDI, une analyse de lysats de promastigotes de GLC94 dénaturés en présence de fortes concentrations de DTT (0.5mM) a été effectuée. Dans ces conditions, l'anti-LmPDI ne détecte plus de protéines à 105 kDa. Ces résultats suggèrent que la LmPDI s'organise en oligomères. La protéine de 35 kDa semble être un contaminant. En effet, l'anti-LmPDI purifié sur colonne d'affinité (Sepharose 4B-LmPDI) ne reconnaît plus la protéine de 35 kDa (figure 6).

Afin de comparer le niveau d'expression de la LmPDI entre les isolats les plus virulents et les moins virulents, les protéines de promastigote ont été extraites puis quantifiées en phase stationnaire de croissance. 5µg de protéines ont été analysés sur un gel à12% de polyacrylamide-SDS et transférés sur une membrane de nitrocellulose. L'analyse par Western blot à l'aide de l'anticorps anti-LmPDI montre que la LmPDI (55kDa) et son dimère (105kDa) sont plus fortement exprimés dans les isolats les plus virulents (figure 7). En revanche, la protéine contaminante de 35kDa est exprimée de manière équivalente quelle que soit la souche testée. Ces résultats suggèrent une corrélation entre le niveau d'expression de la LmPDI et le pouvoir pathogène des isolats étudiés.

### Exemple 3 : Induction par la LmPDI de la prolifération in vitro de cellules mononucléées d'individus ayant des lésions actives ou des antécédents de LCZ

La LmPDI de *L. major,* vu son expression importante au cours du stade infectieux du parasite, pourrait être la cible d'une réponse immune cellulaire. Afin de vérifier la pertinence de cette hypothèse, la capacité de la LmPDI à induire une réponse immune cellulaire a été évaluée, par des expériences de prolifération de cellules mononucléées obtenues d'individus ayant des lésions actives ou des antécédents de LCZ.

Cette étude a été réalisée chez 37 individus vivants à El Guettar (sud de la Tunisie) pour lesquels le résultat du test de prolifération cellulaire contre les antigènes totaux du parasite (SLA, test indiquant un contact antérieur avec le parasite) est disponible. Ces individus se répartissent comme suit :
Groupe 1 : composé de 8 individus ayant un test SLA négatif
Groupe 2 : composé de 29 individus ayant un test SLA positif

Les cellules mononucléees, comprenant les lymphocytes et les monocytes sont séparées à partir sang périphérique par centrifugation sur gradient de ficoll /Hypaque (Pharmacia, Uppsala, Sweden).

Le résultat (figure 8) montre une prolifération significative avec les individus immuns.

L'induction des cytokines (IFN-γ IL-4) dans les surnageants de culture de PBMC a été faite par incubation pendant 48 heures des cellules mono-nucléées avec la même concentration de LmPDI et le dosage a été réalisé par un test ELISA utilisant des anticorps monoclonaux anti-IL-4 et anti-IFN-γ humaines (Pharmingen, San Diego, CA).

Les résultats ont été réalisés sur un échantillon faible d'individus. Ils montrent clairement l'absence d'IL-4 et la présence de taux significatifs d'IFN-γ dans le surnageant des cellules stimulées par la LmPDI.

Ce résultat montre une réponse essentiellement de type Th1, indiquant que la LmPDI pourrait constituer un candidat-vaccin contre les leishmanioses.

### Exemple 4 : Inhibition de la croissance de L. major en milieu liquide, en présence d'un inhibiteur des PDI

La bacitracine est un inhibiteur connu des PDI. Des expériences utilisant la bacitracine montrent qu'à la concentration finale de 2 mM, la bacitracine inhibe complètement la croissance des parasites *L. major* en milieu liquide (figure 9).

Ces expériences ont été effectuée dans les conditions expérimentales suivantes :

### a) Préparation de la RNase A réduite et dénaturée :

20mg de Ribonucléase (RNase A) purifiée ont été réduits et dénaturés à température ambiante pendant 18 heures dans un tampon contenant 0.15M de DTT, 6M de guanidine-HCl et 0.1 M de Tris-HCl à pH 8.6, avant d'être purifiés sur une colonne de sephadex G-25 équilibrée dans du 0.01 M HCl. La concentration des fractions de RNase A réduite et dénaturée a été déterminée à l'aide du coefficient d'extinction de 9200 M⁻¹cm⁻¹ à 275nm. Les fractions sont conservées à -80°C pendant deux semaines.

### b) Réactivation de la RNase A en présence de la protéine recombinante LmPDI :

La RNase A réduite et dénaturée (8µM) a été incubée dans un tampon contenant 4.5 mM (cCMP), 1 mM Glutathione GSH, 0.2 mM Glutathione disulfide GSSH, 2 mM EDTA et 100 mM Tris-HCl pH 8 en présence de Sérum albumine bovine (BSA) (1,4µM) comme contrôle négatif, de Protéine Disulfide Isomérase bovine (1,4µM) comme contrôle positif, ou de LmPDI recombinante (1,4µM) pendant 30 minutes à 25°C. La réactivation de la RNase A est déterminée par la mesure de l'activité RNase A à 296 nm toutes les 5 minutes pendant 30 minutes comme précédemment décrit dans la littérature (Lyles and Gilbert 1991).

### c) Tests d'inhibition in-vitro de l'activitité thio-disulfide oxydo-réductase de la LmPDI recombinante par différents inhibiteurs de PDI :

Les conditions d'expériences relatives aux tests d'inhibition de l'activité thio-disulfide oxydo-réductase de la LmPDI recombinante sont strictement identiques à celles décrites dans le paragraphe (Réactivation de la RNase A en présence de la protéine recombinante LmPDI), excepté le fait que les réactions sont réalisées en présence de 0.01 mM, 0.1 mM, 0.5 mM et 2 mM des inhibiteurs de PDI suivants :
- la bacitracine
- la bacitracine de zinc
- le p-chloromercuribenzoic acid (pCMBA)
- l'acide tocinoic.

### d) Inhibition de la croissance des parasites (L. major) en milieu liquide

Dans le but de déterminer l'effet de la bacitracine (BAC), de la bacitracine de zinc (BACZn), du p-chloromercuribenzoic acid (pCMBA) et du le 5,5'-dithiobis(2-nitrobenzoic acid) (DTNB) sur la croissance *in-vitro* des leishmanies en milieu liquide, différentes concentrations d'inhibiteurs cités ci-dessus 0mM, 0.05mM, 0.1 mM, 0.2mM, 0.5mM, 1mM, 1.5mM, 2mM, 2.5mM et 5mM sont additionnés dans du RPMI additionné de 5% se sérum de veau foetal et contenant 2.10⁶/ml de parasites en phase exponentielle de croissance. Les parasites sont incubés à 26°C et comptés toutes les 24 heures pendant 96 heures. Le comptage des parasites est réalisé sur cellules de Mallassez.

### Exemple 5 : Evaluation de molécules inhibitrices de LmPDI dans leurs capacités à supprimer la croissance de Leishmania major

La mise en valeur du rôle de la LmPDI dans la virulence de *Leishmania* permet d'envisager des nouvelles stratégies de recherche de molécules actives pour soigner les leishmanioses. En effet, il est probable que des molécules connues pour leur activités anti-PDI, autres que la bacitracine, soient capables d'inhiber la croissance du parasite. Un exemple de protocole d'évaluation de molécules potentiellement efficaces contre *Leishmania* est présenté ici.

L'évaluation de molécules connues pour leurs activités anti-PDI, ou celles qui seront nouvellement identifiées, peut se faire en trois étapes. Au cours de la première étape, les molécules sont testées *in vitro* sur la protéine LmPDI recombinante produite dans *Esherichia (E.) coli.* Ensuite, des tests d'inhibition de la croissance des parasites en milieu liquide sont réalisés et enfin, les molécules sont testées dans le modèle murin de la leishmaniose expérimentale.
1- Evaluation de l'inhibition de la LmPDI recombinante. La technique détaillée de l'analyse de l'activité PDI de LmPDI est décrite dans l'exemple 2 et dans les Matériels et Méthodes ci-dessus. La même technique peut être utilisée pour évaluer la capacité qu'ont certains inhibiteurs des PDI connus ou à identifier (en utilisant un modèle moléculaire de la LmPDI), en rajoutant dans le volume réactionnel différentes concentration d'inhibiteurs potentiels. Les résultats sont exprimés en pourcentage d'inhibition par rapport au tampon seul. Ne sont retenues que les molécules qui ont une activité inhibitrice de LmPDI significative et dose-dépendante.
   Dans le but de déterminer si les différents inhibiteurs de PDI décrits dans la littérature pouvaient inhiber l'activité thio-disulfide oxydo-réductase de la LmPDI, , la capacité de ces inhibiteurs à bloquer l'activité enzymatique de la LmPDI synthétisée chez *E. coli* puis purifiée a été étudiée dans un test *in-vitro,* à différentes concentrations. Les inhibiteurs sont :
   - la bacitracine
   - la bacitracine de zinc
   - le p-chloromercuribenzoic acid (pCMBA)
   - l'acide tocinoïque.

   Les résultats obtenus sont représentés dans la figure 10 et montrent que l'activité LmPDI est complètement inhibée en présence de 0.01 mM de pCMBA et de 2mM de bacitracine ou de bacitracine de zinc. En revanche, l'acide tocinoïque ne semble pas avoir un effet très important sur l'activité de la LmPDI aux concentrations utilisées (concentrations qui inhibent totalement l'activité de la PDI humaine).
2- Inhibition de la croissance des parasites (*L. major)* en milieu liquide. La molécule à tester est dissoute dans le solvant approprié, selon ses propriétés physico-chimiques (solubilité dans les solutions aqueuses ou les solvant organiques). Dans tous les cas, le solvant seul est utilisé comme contrôle. Les expériences peuvent, par exemple, être réalisées sur l'isolat *L. major* GLC94. La composition du milieu de culture est détaillée ci-dessus (exemple 2, et Matériels et Méthodes). Les cultures sont incubées à 26°C et repiquées régulièrement pour maintenir les parasites en phase stationnaire de croissance. Pour certaines expériences, le stade amastigote-like du parasite est utilisé. Dans ce cas, les parasites (promastigotes) de la phase stationnaire de croissance sont centrifugés, le milieu est remplacé par le Schneider Drosophilia medium ajusté à pH 5.0 et additionné de 15% de sérum de veau foetal (SVF). Les cultures sont ensuite incubées sous 5% de CO₂ à 35°C.
   L'inhibition de la croissance des promastigotes de *L. major* se fait sur les parasites pris en phase de croissance exponentielle, ajustés à la concentration initiale de 10⁶ parasites/ml de milieu complet et incubés à raison de 100 µl / puits dans les plaques de cultures de 96 puits en l'absence ou en présence de différentes concentrations de la molécule à tester. Les parasites sont incubés sous 5% de CO₂ à 26°C et comptés toutes les 24h pendant 96h. Le comptage des parasites se fait sur cellule de Mallassez. Alternativement, un hémocytomètre peut être utilisé. Toutes les mesures sont faites en triplicata. La capacité inhibitrice d'une molécule est appréciée en concentration inhibitrice qui réduit la division cellulaire par 50% par rapport au contrôle (IC50).
   L'évaluation de la réduction de la viabilité des amastigotes se fait grâce à un test fluorométrique utilisant Almar Blue comme indicateur de viabilité/croissance.
   Les inhibiteurs de PDI décrits dans le paragraphe (1-) ont été testés dans le but d'évaluer leurs capacités à inhiber la croissance *in-vitro* des parasites. Pour cela, différentes quantités d'inhibiteurs sont additionnés dans du RPMI contenant 2.10⁶/ml de parasites en phase exponentielle de croissance. Les parasites sont incubés à 26°C et comptés toutes les 24 heures pendant 96 heures. Le comptage des parasites est réalisé sur cellules de Mallassez. Les résultats obtenus sont représentés dans la figure 11 et montrent que la bacitracine, la bacitracine de zinc ou le pCMBA inhibent entièrement la croissance des leishmanies aux concentrations 5 mM et 2 mM et 0,5 mM respectivement. En revanche, le 5,5'-dithiobis(2-nitrobenzoic acid) (DTNB) aux concentrations utilisées (concentrations qui inhibent totalement l'activité de la PDI humaine) ne semble pas avoir un effet très important sur la croissance des leishmanies.
3- Evaluation de l'efficacité des inhibiteurs pré sélectionnés dans le modèle expérimental de l'infection des souris sensibles BALB/c par *L. major.* Les expérience *in vivo* vont dépendre de la toxicité et des propriétés physico-chimiques des molécules à tester. Les souris BALB/c seront infectées par 10⁶ promastigotes de *L. major* obtenus durant la phase stationnaire de croissance et injectés (dans un volume de 50µl) dans le coussinet plantaire de la patte arrière droite. La mesure du diamètre des lésions se fera de façon hebdomadaire grâce à l'utilisation d'un pied à coulisse.
   En tout, trois protocoles thérapeutiques seront appliqués selon les cas :
   - Pour les molécules hydrophobes, qui diffusent bien, peu ou pas toxiques, le produit sera injecté à différentes concentrations et selon différents schémas par voie intra-péritonéale. La fréquence des injections dépendra de la bio-disponibilité de la molécule ainsi que sa demi-vie. Dans tous les cas, le protocole sera arrêté au bout de 9 semaines après infection.
   - Pour les molécules hydrosolubles et relativement toxiques, les injections se feront en intra-lésionnel (en général, les doses actives peuvent être divisées par 10), par au moins quatre injections au niveau de la zone indurée.
   - Pour les molécules liposolubles, une pommade sera testée par application quotidienne sur la lésion expérimentale.

   Globalement, et quelle que soit la voie d'injection du produit à tester, deux types de protocoles seront effectués :
   - un protocole qui démarre immédiatement après injection des parasites,
   - un protocole qui démarre 4 à 5 semaine après injection des parasites, à un moment où la lésion sera déjà établie.

Dans tous les cas, à la fin du protocole, les souris seront sacrifiées, une estimation de la charge parasitaire se fera au niveau du site d'injection et dans le ganglion qui draine la lésion.

### Exemple 6 : Infection in vitro de macrophages murins par Leishmania

Des macrophages murins de moelle osseuse (*murine bone marrow macrophages,* ou MBMM) sont obtenus à partir de moelle osseuse extrudée d'un fémur ou d'un tibia de souris BALB/c femelles.Les MBMM sont cultivées en plaques multi-chambres à 1,5 x 10³ cellules par puits dans 500 µl de milieu complet. Pour stimuler la croissance et la maturation des MBMM, le milieu de culture est supplémenté avec 20% de milieu conditionné avec des fibroblastes L-929, comme source de facteur stimulateur de colonie de macrophages (*macrophage colony stimulating factor,* ou *MCSF*). Après 6 jours de culture à 37°C et 5% CO₂, le milieu est enlevé, les MBMM sont lavées, et du milieu RPMI frais avec 10% de sérum de veau foetal, mais ne comportant pas de milieu conditionné par des fibroblastes L-929, est ajouté. Des amastigotes intra-lésionnels sont purifiés à partir de lésions non-ulcérées, par centrifugation différentielle, et comptés en utilisant le colorant viral bleu trypan. Ces parasites sont utilisés pour infecter les MBMM à un ratio final nde quatre parasites par macrophage. Deux heures après l'addition des parasites, les macrophages sont rincés cinq fois avec du PBS pour éliminer les amastigotes non phagocytés. Les cultures sont ensuite incubées à 37°C dans 95% d'air et 5% de CO₂. Les expériences sont effectuées à différents points dans le temps : 30 minutes et 2, 24 et 72 heures. Aux temps indiqués, les puits sont rincés avec du PBS, les couvercles sont enlevés, et les macrophages infectés sont fixés à l'éthanol pendant 1 heure à température ambiante. Les plaques sont ensuite lavées et colorées au Giemsa pour suivre l'infection.

L'énumération des macrophages infectés est réalisée au centre de chaque puits, où les cellules sont bien étalées et où les parasites peuvent être facilement énumérés. A ce niveau de la plaque, le ratio parasite/macrophage peut être supérieur à 4.

### Exemple 7: Inhibition de l'activité enzymatique de la LmPDI recombinante par des inhibiteurs de Protéines Disulfide Isomérases

Plusieurs inhibiteurs de Protéines Disulfide Isomérases (PDI) ont été décrits dans la littérature (Ryser *et al.* 1994, Orlandi 1997, Mou *et al.* 1998). Parmi ceux-ci, la bacitracine et la bacitracine de zinc constituent un complexe d'antibiotiques polypeptidiques produits par *Bacillus subtilis* et *Bacillus licheniformis.* La bacitracine A est le composé principal de la bacitracine commerciale, qui est un mélange d'au moins neuf bacitracines. Cet antibiotique est capable d'inhiber la synthèse de la paroi de nombreuses bactéries Gram+, mais également l'activité de plusieurs protéases telles que PDI, transglutaminase, papaïne et neuropeptidase. La plupart de ces protéases ont un résidu cystéine dans leur site actif.

Dans un premier temps, les inventeurs ont testé l'effet de ces inhibiteurs pour vérifier leur capacité éventuelle à altérer l'activité enzymatique de la LmPDI recombinante (rLmPDI) *in vitro.*

La technique de la *scrambled* RNase (RNase réduite et dénaturée), décrite précédemment (Lyles et Gilbert, 1991), a été utilisée pour mettre en évidence l'activité de la LmPDI. Vingt milligrammes de RNase A (Amersham-Pharmacia) ont été dénaturés dans un tampon composé de 0,15M Dithiothréitol, 6M Guanidine HCl et 0,1 M Tris-CI pH8,6 pendant 18 heures à température ambiante. La RNase dénaturée et réduite a énsuite été purifiée sur une colonne de Séphadex G25 équilibrée dans du HCl 0,01 M et quantifiée par spectrophotométrie à 275nm.

Dans un tampon réducteur à base de glutathione, la PDI catalyse la renaturation de la RNase réduite et dénaturée (Gilbert 1998). La restauration de l'activité RNase a été mesurée par spectrophotométrie en présence de cytidine 2'-3'-cyclic monophosphate (cCMP) comme substrat. Ainsi, 8µM-de-RNase-réduite et dénaturée seule ou en présence de 1,4 µM d'albumine bovine (BSA) ou de 1,4 µM de rLmPDI ont été mélangés dans un tampon contenant 4,5 mM cCMP, 1 mM de glutathione réduite (GSH), 200 µM de glutathione oxydée (GSSG), 2 mM EDTA et 100 mM Tris-CI pH8. La réaction s'est effectuée à température ambiante pendant 30 minutes. L'hydrolyse du cCMP résultant de la renaturation de la RNase a été enregistrée par la mesure de l'absorbance à 296 nm toutes les 5 minutes pendant la demi-heure de réaction.

L'activité de la LmPDI recombinante (rLmPDI) a été mesurée en présence de différentes concentrations de bacitracine (BAC 0,01 mM-2 mM) et de bacitracine de zinc (BACZn 0,01 mM-2 mM). Les résultats sont montrés à la figure 12.

Ces résultats montrent que la bacitracine et la bacitracine de zinc ont des effets similaires. Ainsi, en présence de ces deux produits, 50% d'inhibition est observée à 0,1 mM, 70% à 0,5 mM et 100% à 2 mM. Les concentrations qui inhibent la rLmPDI sont comparables à celles décrites dans la littérature comme inhibitrices de PDI d'autres espèces.

### Exemple 8 : Cinétique de croissance in vitro des promastigotes de L. major en présence de Bacitracine de Zinc

Les inventeurs ont ensuite testé l'effet de la bacitracine de zinc sur la cinétique de croissance *in vitro* de promastigotes de *L. major.* Pour cette étude, seule la bacitracine de zinc a été testée, d'une part parce qu'elle présente le même profil d'inhibition de l'activité enzymatique de la rLmPDI que la bacitracine, et d'autre part, parce que la bacitracine est plus stable et moins toxique lorsqu'elle est couplée au zinc.

Pour ce faire, les promastigotes de l'isolat GLC94 ont été mis en culture sur du milieu à base de sérum de lapin coagulé pendant deux jours. Par la suite, les parasites (2x10⁶ parasites par ml) ont été transférés dans du milieu complet comprenant la bacitracine de zinc BACZn, à 1 ; 1,5 et 2,5 mM. Des promastigotes cultivés dans du milieu complet en l'absence d'inhibiteurs ont été utilisés comme contrôle. Le suivi s'est fait par comptage des parasites à 48, 72 et 96 heures. Les résultats sont montrés à la figure 13.

Ces résultats montrent que la bacitracine de zinc inhibe partiellement la croissance des parasites à 1,5 mM et totalement à 2 mM et à 5 mM, alors qu'elle n'a aucun effet à 1 mM. Il est donc très intéressant de noter que cette molécule est capable de bloquer la prolifération de parasites *L. major* en culture.

### Exemple 9 : Inhibition de la croissance des promastigotes de L. major chez la souris BALS/c, en présence de Bacitracine de Zinc

La disponibilité de la bacitracine de zinc, qui fait déjà partie de l'arsenal thérapeutique, a permis de tester son effet sur l'évolution de l'infection de souris BALB/c par *L. major.* Ainsi, des souris ont été infectées avec des promastigotes en phase stationnaire de croissance (10⁶ promastigotes par patte) de l'isolat GLC94 dans le coussinet plantaire de la patte arrière et traitées avec une pommade à base de 5 mM ou 25 mM de BACZn (préparées dans de la vaseline). Le traitement par la pommade a été démarré 48 heures après injection des parasites, à raison d'une application par jour pendant 5 jours par semaine. Des souris infectées de la même manière et traitées avec de la vaseline ont été utilisées comme contrôle. La taille des lésions a été mesurée chaque semaine. Les résultats sont montrés dans la figure 14.

Bien que préliminaires, ces résultats montrent que la bacitracine de zinc atténue la progression de la maladie lorsqu'elle est appliquée localement sous forme de pommade, au niveau du site d'injection des souris BALB/c. Il est à souligner que dans le groupe des souris traitées, l'atténuation de la lésion est observée chez 2 sur les 3 souris traitées avec de la bacitracine 5 mM et 2 sur les 4 souris traitées avec de la bacitracine 25 mM. La reprise de la maladie clinique après arrêt du traitement, est attendue dans la mesure où les souris BALB/c sont incapables d'éliminer totalement le parasite et que même les traitements utilisés chez l'homme (Glucantime et paramomycine) ont peu d'effets sur la maladie induite chez la souris BALB/c, chez qui la disparition totale des parasites n'a jamais été observée.

La LmPDI peut donc être considérée comme une cible potentielle de chimiothérapie anti-leishmanienne, et il apparaît que la bacitracine est potentiellement efficace contre *L. major.*

### BIBLIOGRAPHIE

Beverley, S. M. and S. J. Turco (1998). "Lipophosphoglycan (LPG) and the identification of virulence genes in the protozoan parasite Leishmania." Trends Microbiol 6(1): 35-40.
Chakrabarty, R., S. Mukherjee, et al. (1996). "Kinetics of entry of virulent and avirulent strains of Leishmania donovani into macrophages: a possible role of virulence molecules (gp63 and LPG)." J Parasitol 82(4): 632-5.
Cotrim, P. C., L. K. Garrity, et al. (1999). "Isolation of genes mediating résistance to inhibitors of nucleoside and ergosterol metabolism in Leishmania by overexpression/selection." J Biol Chem 274(53): 37723-30.
De, T. and S. Roy (1999). "Infectivity and attenuation of Leishmania donovani promastigotes: association of galactosyl transferase with loss of parasite virulence." J Parasitol 85(1): 54-9.
Descoteaux, A., Y. Luo, et al. (1995). "A specialized pathway affecting virulence glycoconjugates of Leishmania." Science 269(5232): 1869-72.
Desjardins, M. and A. Descoteaux (1997). "Inhibition of phagolysosomal biogenesis by the Leishmania lipophosphoglycan." J Exp Med 185(12): 2061-8.
Desjardins, M. and A. Descoteaux (1998). "Survival strategies of Leishmania donovani in mammalian host macrophages." Res Immunol 149(7-8): 689-92.
Dumas, C., M. Ouellette, et al. (1997). "Disruption of the trypanothione reductase gene of Leishmania decreases its ability to survive oxidative stress in macrophages." Embo J 16(10): 2590-8.
Ferrari, D. M. and H. D. Soling (1999). "The protein disulphide-isomerase family: unravelling a string of folds." Biochem J 339(Pt 1): 1-10.
Frand, A. R., J. W. Cuozzo, et al. (2000). "Pathways for protein disulphide bond formation." Trends Cell Biol 10(5): 203-10.
Garami, A. and T. llg (2001). "The role of phosphomannose isomerase in Leishmania mexicana glycoconjugate synthesis and virulence." J Biol Chem 276(9): 6566-75.
Gilbert, H. F. (1998). "Protein disulfide isomerase." Methods Enzymol 290: 26-50.
Heard, P. L., C. S. Lewis, et al. (1996). "Leishmania mexicana amazonensis: differential display analysis and cloning of mRNAs from attenuated and infective forms." J Eukaryot Microbiol 43(5): 409-15.
Hubel, A., S. Krobitsch, et al. (1997). "Leishmania major Hsp100 is required chiefly in the mammalian stage of the parasite." Mol Cell Biol 17(10): 5987-95.
Hultgren, S. J., S. Abraham, et al. (1993). "Pilus and nonpilus bacterial adhesins: assembly and function in cell récognition." Cell 73(5): 887-901.
IIg, T. (2000). "Proteophosphoglycans of Leishmania." Parasitol Today 16(11): 489-97.
IIg, T., M. Demar, et al. (2001). "Phosphoglycan Repeat-deficient Leishmania mexicana Parasites Remain Infectious to Macrophages and Mice." J Biol Chem 276(7): 4988-97.
Kebaïer, C., H. Louzir, et al. (2001). "Heterogeneity of wild Leishmania major isolates in experimental murine pathogenicity and specific immune response." Infection and Immunity 69(8).
Khalil, E. A., A. M. El Hassan, et al. (2000). "Autoclaved Leishmania major vaccine for prévention of visceral leishmaniasis: a randomised, double-blind, BCG-controlled trial in Sudan." Lancet 356(9241): 1565-9.
Liang, P., D. Bauer, et al. (1995). "Analysis of altered gene expression by differential display." Methods Enzymol 254: 304-21.
Liang, P. and A. B. Pardee (1992). "Differential display of eukaryotic messenger RNA by means of the polymerase chain reaction." Science 257(5072): 967-71.
Lira, R., S. Sundar, et al. (1999). "Evidence that the high incidence of treatment failures in Indian kala- azar is due to the emergence of antimony-resistant strains of Leishmania donovani." J Infect Dis 180(2): 564-7.
Louzir, H., P. C. Melby, et al. (1998). "Immunologic déterminants of disease evolution in localized cutaneous leishmaniasis due to Leishmania major." J Infect Dis 177(6): 1687-95.
Lyles, M. M. and H. F. Gilbert (1991). "Catalysis of the oxidative folding of ribonuclease A by protein disulfide isomerase: dependence of the rate on the composition of the redox buffer." Biochemistry 30(3): 613-9.
Lyles, M. M. and H. F. Gilbert (1991). "Catalysis of the oxidative folding of ribonuclease A by protein disulfide isomerase: pre-steady-state kinetics and the utilization of the oxidizing equivalents of the isomerase." Biochemistry 30(3): 619-25.
Martin, J. L. (1995). "Thioredoxin--a fold for ail reasons." Structure 3(3): 245-50.
McKerrow, J. H., J. C. Engel, et al. (1999). "Cysteine protease inhibitors as chemotherapy for parasitic infections." Bioorg Med Chem 7(4): 639-44.
Mottram, J. C., D. R. Brooks, et al. (1998). "Roles of cysteine proteinases of trypanosomes and Leishmania in host- parasite interactions." Curr Opin Microbiol 1(4): 455-60.
Mottram, J. C., A. E. Souza, et al. (1996). "Evidence from disruption of the Imcpb gene array of Leishmania mexicana that cysteine proteinases are virulence factors." Proc Natl Acad Sci U S A 93(12): 6008-13.
Mou, Y., H. Ni, et al. (1998). "The selective inhibition of beta 1 and beta 7 integrin-mediated lymphocyte adhésion by bacitracin." J Immunol 161(11): 6323-9.
Mukhopadhyay, S., P. Sen, et al. (1998). "Reduced expression of lipophosphoglycan (LPG) and kinetoplastid membrane protein (KMP)-11 in Leishmania donovani promastigotes in axenic culture." J Parasitol 84(3): 644-7.
Noiva, R. (1999). "Protein disulfide isomerase: the multifunctional redox chaperone of the endoplasmic reticulum." Semin Cell Dev Biol 10(5): 481-93.
Orlandi, P. A. (1997). "Protein-disulfide isomerase-mediated réduction of the A subunit of cholera toxin in a human intestinal cell line." J Biol Chem 272(7): 4591-9.
Ostermeier, M., K. De Sutter, et al. (1996). "Eukaryotic protein disulfide isomerase complements Escherichia coli dsbA mutants and increases the yield of a heterologous secreted protein with disulfide bonds." J Biol Chem 271(18): 10616-22.
Paramchuk, W. J., S. O. Ismail, et al. (1997). "Cloning, characterization and overexpression of two iron superoxide dismutase cDNAs from Leishmania chagasi: role in pathogenesis." Mol Biochem Parasitol 90(1): 203-21.
Peek, J. A. and R. K. Taylor (1992). "Characterization of a periplasmic thiol:disulfide interchange protein required for the functional maturation of secreted virulence factors of Vibrio cholerae." Proc Natl Acad Sci U S A 89(13): 6210-4.
Perez-Victoria, J. M., F. J. Perez-Victoria, et al. (2001). "High-affinity binding of silybin derivatives to the nucleotide-binding domain of a Leishmania tropica P-glycoprotein-like transporter and chemosensitization of a multidrug-resistant parasite to daunomycin." Antimicrob Agents Chemother 45(2): 439-46.
Ryser, H. J., E. M. Levy, et al. (1994). "Inhibition of human immunodeficiency virus infection by agents that interfere with thiol-disulfide interchange upon virus-receptor interaction." Proc Natl Acad Sci U S A 91(10): 4559-63.
Ryan, K. A., L. A. Garraway, et al. (1993). "Isolation of virulence genes directing surface glycosyl- phosphatidylinositol synthesis by functional complementation of Leishmania." Proc Natl Acad Sci U S A 90(18): 8609-13.
Sacks, D. L., G. Modi, et al. (2000). "The role of phosphoglycans in Leishmania-sand fly interactions." Proc-Natl Acad Sci U S A 97(1): 406-11.
Selzer, P. M., X. Chen, et al. (1997). "Leishmania major: molecular modeling of cysteine proteases and prédiction of new nonpeptide inhibitors." Exp Parasitol 87(3): 212-21.
Sharifi, I., A. R. FeKri, et al. (1998). "Randomised vaccine trial of single dose of killed Leishmania major plus BCG against anthroponotic cutaneous leishmaniasis in Bam, Iran." Lancet 351(9115): 1540-3.
Spath, G. F., L. Epstein, et al. (2000). "Lipophosphoglycan is a virulence factor distinct from related glycoconjugates in the protozoan parasite Leishmania major." Proc Natl Acad Sci U S A 97(16): 9258-63.
Streit, J. A., T. J. Recker, et al. (2001). "Protective immunity against the protozoan Leishmania chagasi is induced by subclinical cutaneous infection with virulent but not avirulent organisms." J Immunol 166(3): 1921-9.
Titus, R. G., F. J. Gueiros-Filho, et al. (1995). "Development of a safe live Leishmania vaccine line by gene replacement." Proc Natl Acad Sci U S A 92(22): 10267-71.
Wang, Y., E. S. Bjes, et al. (2000). "Molecular aspects of complement-mediated bacterial killing. Periplasmic conversion of C9 from a protoxin to a toxin." J Biol Chem 275(7): 4687-92.
Wiese, M. (1998). "A mitogen-activated protein (MAP) kinase homologue of Leishmania mexicana is essential for parasite survival in the infected host." Embo J 17(9): 2619-28.
Yu, J. (1998). "Inactivation of DsbA, but not DsbC and DsbD, affects the intracellular survival and virulence of Shigella flexneri." Infect Immun 66(8): 3909-17.
Yu, J., B. Edwards-Jones, et al. (2000). "Key rôle for DsbA in cell-to-cell spread of Shigella flexneri, permitting secretion of Ipa proteins into interepithelial protrusions." Infect Immun 68(11): 6449-56.
Yu, J. and J. S. Kroll (1999). "DsbA: a protein-folding catalyst contributing to bacterial virulence." Microbes Infect 1(14): 1221-8.
Yu, J., H. Webb, et al. (1992). "A homologue of the Escherichia coli DsbA protein involved in disulphide bond formation is required for enterotoxin biogenesis in Vibrio cholerae." Mol Microbiol 6(14): 1949-58.
Zhang, H. Z. and M. S. Donnenberg (1996). "DsbA is required for stability of the type IV pilin of enteropathogenic escherichia coli." Mol Microbiol 21(4): 787-97.
Zhang, W. W. and G. Matlashewski (1997). "Loss of virulence in Leishmania donovani deficient in an amastigote- specific protein, A2." Proc Natl Acad Sci U S A 94(16): 8807-11.

## Revendications

1. Protéine de *Leishmania* impliquée dans la virulence du parasite, comportant au moins un site (Cys-Gly-His-Cys) identique au site actif potentiel d'une protéine de la famille des protéines disulfides isomérases (PDI), ladite protéine de *Leishmania* étant la protéine de SEQ ID NO : 2.

2. Variant fonctionnel de la protéine selon la revendication 1, présentant au moins 80% d'identité avec celle-ci.

3. Polypeptide recombinant comportant au moins un fragment de plus de 10 acides aminés d'une protéine selon l'une quelconque des revendications 1 à 2, ledit polypeptide recombinant étant susceptible de déclencher une réaction immunologique contre un épitope de la protéine selon la revendication 1, lorsqu'il est administré à un hôte, humain ou animal.

4. Polypeptide recombinant selon la revendication 3, **caractérisé en ce qu'**il s'agit de la protéine LmPDI-(His)₆ de séquence SEQ ID No : 3.

5. Protéine de fusion comportant un polypeptide recombinant selon la revendication 3, fusionné à un autre fragment polypeptidique, ladite protéine de fusion étant susceptible de déclencher une réaction immunologique contre un épitope de la protéine selon la revendication 1, lorsqu'elle est administrée à un hôte, humain ou animal.

6. Acide nucléique, recombinant **caractérisé en ce que** sa séquence code une protéine ou un polypeptide selon l'une quelconque des revendications 1 à 5, sous réserve que la séquence de cet acide nucléique ne soit pas celle du contig 2285105-2298564 disponible sous le numéro d'accession AL499624.2.

7. Séquence d'acide nucléique selon la revendication 6, **caractérisée en ce qu'**elle comprend la séquence codante correspondant aux nucléotides 241 à 1674 de la séquence SEQ ID No: 1, ou un fragment de cette séquence de taille supérieure ou égale à 100 nucléotides.

8. Vecteur d'acide nucléique, **caractérisé en ce qu'**il comprend une séquence d'acide nucléique selon la revendication 6 ou 7.

9. Vecteur selon la revendication 8, **caractérisé en ce qu'**il s'agit d'un plasmide, d'un cosmide, d'un phage, ou d'un virus.

10. Cellule en culture comprenant un vecteur selon la revendication 8 ou 9.

11. Cellule selon la revendication 10, **caractérisée en ce qu'**il s'agit de la souche bactérienne LmPDI-XL₁, déposée à la Collection Nationale de Culture des Microorganismes (CNCM) le 31/01/2001 sous le numéro I-2621.

12. Utilisation d'une sonde d'acide nucléique s'hybridant spécifiquement, en conditions de forte stringence, avec la séquence nucléique de SEQ ID No: 1, pour déterminer la présence ou l'absence du gène de virulence codant pour la protéine de la revendication 1, dans un échantillon biologique.

13. Amorce nucléotidique **caractérisée en ce qu'**elle permet l'amplification spécifique d'au moins un fragment de la séquence de SEQ ID No : 1, à partir de cellules infectées par *Leishmania major,* permettant ainsi de déterminer la présence ou l'absence du gène de virulence codant la protéine de SEQ ID No : 2 (LmPDI), dans un échantillon biologique.

14. Anticorps purifié, reconnaissant spécifiquement la protéine de la revendication 1.

15. Composition immunogène, comportant une protéine selon la revendication 1, 2 ou 5 et/ou un polypeptide recombinant selon la revendication 3 ou 4, et/ou un acide nucléique recombinant dont la séquence code une telle protéine, et/ou un acide nucléique recombinant dont la séquence code un tel polypeptide, et/ou un acide nucléique selon la revendication 7, et/ou un vecteur d'acide nucléique comprenant un tel acide nucléique, et/ou une cellule comprenant un tel vecteur, ladite composition immunogène étant capable de stimuler *in vitro* la prolifération de cellules mononucléées provenant d'individus ayant été en contact avec un parasite *Leishmania.*

16. Composition immunogène selon la revendication 15, capable de stimuler *in vitro* la prolifération de cellules mononucléées provenant d'individus ayant été en contact avec *Leishmania major.*

17. Composition immunogène selon la revendication 15 ou 16, de formulation pharmaceutiquement acceptable pour être administrée à un hôte humain ou animal.

18. Composition immunogène selon l'une quelconque des revendications 15 à 17, capable d'induire une réponse immunitaire de type Th1 lorsqu'elle est administrée à un hôte, humain ou animal.

19. Composition vaccinante comportant une protéine selon la revendication 1, 2 ou 5, et/ou un polypeptide recombinant selon la revendication 3 ou 4, et/ou un acide nucléique recombinant dont la séquence code une telle protéine, et/ou un acide nucléique recombinant dont la séquence code un tel polypeptide, et/ou un acide nucléique selon la revendication 7, et/ou un vecteur d'acide nucléique comprenant un tel acide nucléique, et/ou une cellule comprenant un tel vecteur, ladite composition vaccinante étant destinée à protéger un hôte, humain ou animal, contre la leishmaniose.

20. Composition vaccinante selon la revendication 19, de formulation pharmaceutiquement acceptable pour être administrée à une hôte humain ou animal.

21. Composition immunogène et/ou vaccinante selon l'une quelconque des revendications 15 à 20, comportant en outre un antigène étranger à *Leishmania* et/ou une séquence d'acide nucléique codant pour un antigène étranger à *Leishmania.*

22. Procédé de criblage *in vitro* de molécules susceptibles d'inhiber la croissance de *Leishmania major,* comportant une étape d'évaluation de la capacité desdites molécules à inhiber l'activité de la protéine de la revendication 1.

23. Procédé de criblage selon la revendication 22, dans lequel l'étape d'évaluation de la capacité d'une molécule à inhiber l'activité de la protéine de la revendication 1 est effectuée dans un test de réactivation de la RNase A réduite et dénaturée, comportant les étapes suivantes :
- incubation de la RNase A réduite et dénaturée en présence de la protéine de la revendication 1, dans des conditions permettant sa réactivation,
- incubation de la RNase A réduite et dénaturée dans des conditions identiques à celles permettant sa réactivation par la protéine de la revendication 1, en ajoutant la molécule à tester,
- comparaison des résultats obtenus en absence et en présence de la molécule à tester, un défaut de réactivation de la RNase A en présence de la molécule testée révélant que cette molécule possède une activité inhibitrice à l'égard de la protéine de la revendication 1.

24. Procédé de criblage selon les revendications 22 et 23, comportant en outre un test d'inhibition de la croissance de *Leishmania major* en milieu liquide.

25. Utilisation d'un ou plusieurs composé(s) qui inhibe(nt) une Protéine Disulfide Isomérase (PDI) de *Leishmania major,* pour la préparation d'une composition pharmaceutique destinée à la prophylaxie, à l'atténuation, ou au traitement d'une infection par *Leishmania major.*

26. Utilisation selon la revendication 25, dans laquelle ledit composé inhibiteur ou un desdits composés inhibiteurs est un anticorps anti-PDI ou anti-LmPDI, la bacitracine, la bacitracine de zinc, l'acide 5,5'-dithiobis(2-nitrobenzoïque) (DTNB), l'acide p-chloromercuribenzenesulfonique (pCMBS), ou l'acide tocinoïque.

27. Utilisation selon la revendication 25 ou 26, pour la préparation d'une composition administrable à un hôte humain ou animal, par voie topique, orale ou parentérale.

28. Utilisation de la bacitracine ou de la bacitracine de zinc pour la préparation d'une composition pharmaceutique destinée à inhiber la croissance d'un parasite responsable de la leishmaniose ou comme agent actif contre une infection à *Leishmania.*

29. Composition pharmaceutique destinée au traitement d'une infection par *Leishmania,* comportant un anticorps selon la revendication 14.

30. Composition selon la revendication 29, appropriée à une administration topique, orale ou parentérale.

31. Composition pharmaceutique pour le traitement d'une infection par *Leishmania,* contenant un ou plusieurs inhibiteurs des Protéines Disulfides Isomérases (PDI).

32. Composition pharmaceutique selon la revendication 31, contenant de la bacitracine ou de la bacitracine de zinc.

33. Méthode de diagnostic *in vitro* d'une infection par un parasite responsable de la Leishmaniose, **caractérisée en ce qu'**elle comprend :
- la mise en contact d'au moins un anticorps selon la revendication 14 avec un prélèvement biologique d'un sujet partiellement infecté par un parasite responsable de la Leishmaniose, dans des conditions permettant la formation d'un complexe immun entre ledit anticorps et les protéines antigéniques contenues dans le prélèvement,
- la détection dudit complexe.

34. Trousse de diagnostic pour la mise en oeuvre de la méthode selon la revendication 33 **caractérisée en ce qu'**elle comprend :
- au moins un anticorps selon la revendication 14,
- un milieu approprié à la formation d'un complexe immun avec ledit anticorps,
- des réactifs permettant la détection des complexes éventuellement formés,
- le cas échéant, des échantillons témoins.

## Patentansprüche

1. Leishmania-Protein, enthalten in der Parasitenvirulenz, umfassend wenigstens ein (Cys-Gly-His-Cys)-Zentrum, identisch mit einem möglichen aktiven Zentrum eines Proteins der Familie der Protein-Disulfid-Isomerasen (PDI), wobei das Leishmania-Protein ein Protein von SEQ-ID-Nr.: 2 ist.

2. Funktionelle Variante des Proteins gemäß Anspruch 1, welche wenigstens 80% Identität mit diesem Protein besitzt.

3. Rekombinantes Polypeptid, umfassend wenigstens ein Fragment von mehr als 10 Aminosäuren eines Proteins gemäß irgendeinem der Ansprüche 1 bis 2, wobei das rekombinante Polypeptin dazu geeignet ist, eine immunologische Reaktion gegen ein Epitop des Proteins gemäß Anspruch 1 freizusetzen, wenn es einem menschlichen oder tierischen Wirt verabreicht wird.

4. Rekombinantes Polypeptid gemäß Anspruch 3, **dadurch gekennzeichnet, dass** es sich um das Protein LmPDI-(His)₆ der Sequenz SEQ-ID-Nr.: 3 handelt.

5. Fusionspeptid, umfassend ein rekombinantes Polypeptid gemäß Anspruch 3, mit einem anderen polypeptitischen Fragment fusioniert, wobei das Fusionsprotein dazu geeignet ist, eine immunologische Reaktion gegen ein Epitop des Proteins gemäß Anspruch 1 freizusetzen, wenn es einem menschlichen oder tierischen Wirt verabreicht wird.

6. Rekombinante Nukleinsäure, **dadurch gekennzeichnet, dass** ihre Sequenz ein Protein oder ein Polypeptid gemäß einem der Ansprüche 1 bis 5 kodiert, unter dem Vorbehalt, dass die Sequenz dieser Nukleinsäure nicht jene der Contig 2285105-2298564, verfügbar unter der Zugangsnummer AL499624.2, ist.

7. Nukleinsäuresequenz gemäß Anspruch 6, **dadurch gekennzeichnet, dass** sie die Kodierungssequenz entsprechend den Nukleotiden 241 bis 1674 der Sequenz SEQ-ID-Nr.: 1 oder ein Fragment dieser Sequenz in einer Größe von größer oder gleich 100 Nukleotiden umfasst.

8. Nukleinsäurevektor, **dadurch gekennzeichnet, dass** er eine Nukleinsäuresequenz gemäß Anspruch 6 oder 7 umfasst.

9. Vektor gemäß Anspruch 8, **dadurch gekennzeichnet, dass** es sich um ein Plasmid, ein Cosmid, einen Phagen oder einen Virus handelt.

10. Zellkultur, umfassend einen Vektor gemäß Anspruch 8 oder 9.

11. Zelle gemäß Anspruch 10, **dadurch gekennzeichnet, dass** es sich um den Bakterienstamm LmPD1-XL₁ handelt, welcher bei der Collection Nationale de Culture des Microorganismes (CNCM) am 31/01/2001 unter der Nummer 1-2621 hinterlegt wurde.

12. Verwendung einer Nukleinsäuresonde, welche sich unter sehr stringenten Bedingungen spezifisch mit der Nukleinsequenz SEQ-ID-Nr.: 1 hybridisiert, um in einer biologischen Probe das Vorhandensein oder die Abwesenheit eines Virulenzgenes zu bestimmen, das für das Protein gemäß Anspruch 1 kodiert.

13. Nukleotidstarter, **dadurch gekennzeichnet, dass** er eine spezifische Amplifikation wenigstens eines Fragments der Sequenz SEQ-ID-Nr.: 1, ausgehend von durch Leishmania major infizierten Zellen ermöglicht, wodurch so das Vorhandensein oder die Abwesenheit eines Virulenzgenes bestimmt werden kann, welches in einer biologischen Probe das Protein SEQ-ID-Nr.: 2 (LmPDI) kodiert.

14. Gereinigter Antikörper, welcher das Protein von Anspruch 1 spezifisch erkennt.

15. Immunogene Zusammensetzung, umfassend ein Protein gemäß Anspruch 1, 2 oder 5 und/oder ein rekombinantes Polypeptid gemäß Anspruch 3 oder 4 und/oder eine rekombinante Nukleinsäure, deren Sequenz ein solches Protein kodiert, und/oder eine rekombinante Nukleinsäure, deren Sequenz ein solches Peptid kodiert, und/oder eine Nukleinsäure gemäß Anspruch 7, und/oder ein Nukleinsäurevektor, umfassend eine solche Nukleinsäure und/oder eine Zelle, umfassend einen solchen Vektor, wobei die immunogene Zusammensetzung in vitro die Proliferation mononuklearer Zellen stimulieren kann, welche von Individuen abstammen, die mit einem Leishmania-Protein in Kontakt waren.

16. Immunogene Zusammensetzung gemäß Anspruch 15, welche in vitro die Proliferation mononuklearer Zellen stimulieren kann, welche von Individuen stammen, welche mit Leishmania major in Kontakt waren.

17. Immunogene Zusammensetzung gemäß Anspruch 15 oder 16 als pharmazeutisch annehmbare Zusammensetzung zur Verabreichung an einen menschlichen oder tierischen Wirt.

18. Immunogene Zusammensetzung gemäß einem jeden der Ansprüche 15 bis 17, welche eine Immunantwort vom Typ Th1 bewirken kann, wenn sie einem menschlichen oder tierischen Wirt verabreicht wird.

19. Impfzusammensetzung, umfassend ein Protein gemäß Anspruch 1, 2 oder 5 und/oder ein rekombinantes Polypeptid gemäß Anspruch 3 oder 4 und/oder eine rekombinante Nukleinsäure, deren Sequenz ein solches Protein kodiert, und/oder eine rekombinante Nukleinsäure, deren Sequenz ein solches Polypeptid kodiert, und/oder eine Nukleinsäure gemäß Anspruch 7 und/oder einen Nukleinsäurevektor, umfassend eine solche Nukleinsäure, und/oder eine Zelle, umfassend einen solchen Vektor, wobei die Impfzusammensetzung dazu bestimmt ist, einen menschlichen oder tierischen Wirt gegen Leishmaniose zu schützen.

20. Impfzusammensetzung gemäß Anspruch 19 als pharmazeutisch annehmbare Formulierung zur Verabreichung an einen menschlichen oder tierischen Wirt.

21. Immunogene Zusammensetzung und/oder Impfzusammensetzung gemäß einem der Ansprüche 15 bis 20, welche des weiteren ein Fremdantigen auf Leishmania und/oder eine Nukleinsäuresequenz, welche für das Fremdantigen auf Leishmania kodiert, umfasst.

22. Verfahren zum in vitro Klassifizieren von Molekülen, welche dazu geeignet sind, das Wachstum von Leishmania major zu inhibieren, umfassend einen Schritt der Evaluierung der Fähigkeit dieser Moleküle, die Aktivität des Proteins von Anspruch 1 zu inhibieren.

23. Verfahren zur Klassifizierung gemäß Anspruch 22, worin der Schritt der Evaluierung der Fähigkeit eines Moleküls die Aktivität des Proteins von Anspruch 1 zu inhibieren, in einem Reaktivierungstest der reduzierten und denaturierten RNase A bewirkt wird, umfassend die folgenden Schritte:
- Inkubieren der reduzierten und denaturierten RNase A des Proteins von Anspruch 1 unter Bedingungen, welche diese Reaktivierung gestatten,
- Inkubieren der reduzierten und denaturierten RNase A unter Bedingungen, die identisch zu jenen sind, welche die Reaktivierung durch das Protein von Anspruch 1 gestatten, unter Hinzufügen des zu testenden Moleküls,
- Vergleichen der in Abwesenheit und Vorhandensein des zu testenden Moleküls erhaltenen Ergebnisse, wobei ein Fehlen der Reaktivierung der RNase A in Gegenwart des getesteten Moleküls offenbart, dass dieses Molekül eine inhibierende Wirkung gegenüber dem Protein von Anspruch 1 besitzt.

24. Klassifizierungsverfahren gemäß den Ansprüchen 22 und 23, umfassend des weiteren einen Test der Inhibierung des Wachstums von Leishmania major in wässrigem Milieu.

25. Verwendung einer oder mehrerer Verbindungen, welche eine Protein-Disulfid-Isomerase (PDI) von Leishmania major inhibieren, zur Herstellung einer pharmazeutischen Zusammensetzung für die Prophylaxe, Milderung oder Behandlung einer Infektion mit Leishmania major.

26. Verwendung nach Anspruch 25, worin die Inhibitorverbindung oder eine der Inhibitorverbindungen ein anti-PDI- oder anti-LmPDI-Antikörper, Bacitracin, Zink-Bacitracin, 5,5'-Dithiobis-2-Nitrobenzolsäure (DTNB), p-Chlormercurbenzolsulfonsäure (pCMBS) oder Tocinoinsäure ist.

27. Verwendung nach Anspruch 25 oder 26 für die Herstellung einer an einen menschlichen oder tierischen Wirt topisch, oral oder parenteral verabreichbaren Zusammensetzung.

28. Verwendung von Bacitracin oder Zink-Bacitracin zur Herstellung einer pharmazeutischen Zusammensetzung zur Inhibierung des Wachstums eines für Leishmaniose verantwortlichen Parasiten oder als Wirkstoff gegen eine Leishmania-Infektion.

29. Pharmazeutische Zusammensetzung zur Behandlung einer Infektion durch Leishmania, umfassend einen Antikörper gemäß Anspruch 14.

30. Zusammensetzung gemäß Anspruch 29, geeignet zur topischen, oralen oder parenteralen Verabreichung.

31. Pharmazeutische Zusammensetzung zur Behandlung einer Leishmania-Infektion, enthaltend einen oder mehrere Inhibitoren der Protein-Disulfid-Isomerase (PDI).

32. Pharmazeutische Zusammensetzung gemäß Anspruch 31, enthaltend Bacitracin oder Zink-Bacitracin.

33. In vitro Diagnoseverfahren einer Infektion durch einen Parasiten, der für Leishmaniose verantwortlich ist, **dadurch gekennzeichnet, dass** es umfasst:
- das in Kontakt bringen wenigstens eines Antikörpers gemäß Anspruch 14 mit einer biologischen Probe eines Subjekts, das teilweise durch einen für die Leishmaniose verantwortlichen Parasiten infiziert ist, unter Bedingungen, welche die Bildung eines Immunkomplexes zwischen den Antikörpern und den in der Probe enthaltenen antigenen Proteinen erlauben,
- Feststellen des Komplexes.

34. Diagnosekit für die Durchführung des Verfahrens gemäß Anspruch 33 **dadurch gekennzeichnet, dass** es umfasst:
- wenigstens einen Antikörper gemäß Anspruch 14,
- ein für die Bildung eines Immunkomplexes mit dem Antikörper geeignetes Milieu,
- ein Reagenz, das die Feststellung des möglicherweise gebildeten Komplexes erlaubt,
- gegebenenfalls Kontrollproben.

## Claims

1. A Leishmania protein involved in the virulence of the parasite, comprising at least one site (Cys-Gly-His-Cys) identical to the potential active site of a protein from the protein disulfide-isomerase family (PDI), said Leishmania protein being the protein with sequence SEQ ID NO: 2.

2. A functional variant of the protein as claimed in claim 1, having at least 80% identity therewith.

3. A recombinant polypeptide comprising at least one fragment of more than 10 amino acids of a protein as claimed in claim 1 or claim 2, said recombinant polypeptide being capable of triggering an immunological reaction against an epitope of the protein as claimed in claim 1 when administered to a human or animal host.

4. The recombinant polypeptide as claimed in claim 3, **characterized in that** it is the LmPDI-(His)₆ protein with sequence SEQ ID No: 3.

5. A fusion protein comprising a recombinant polypeptide as claimed in claim 3, fused with a further polypeptide fragment, said fusion protein being capable of triggering an immunological reaction against an epitope of the protein as claimed in claim 1 when it is administered to a human or animal host.

6. A recombinant nucleic acid, **characterized in that** its sequence codes for a protein or a polypeptide as claimed in any one of claims 1 to 5, provided that the sequence for this nucleic acid is not that of contig 2285105-2298564 available under accession number AL499624.2.

7. The nucleic acid sequence as claimed in claim 6, **characterized in that** it comprises the coding sequence corresponding to nucleotides 241 to 1674 of sequence SEQ ID No: 1, or a fragment of said sequence 100 nucleotides or more in size.

8. A nucleic acid vector, **characterized in that** it comprises a nucleic acid sequence as claimed in claim 6 or claim 7.

9. The vector as claimed in claim 8, **characterized in that** it is a plasmid, a cosmid, a phage or a virus.

10. A culture cell comprising a vector as claimed in claim 8 or claim 9.

11. The cell as claimed in claim 10, **characterized in that** it is bacterial strain LmPD1-XL₁ deposited at the Collection National de Culture des Microorganismes [CNCM, the National Collection of Microorganism Cultures], on 31/01/2001 with accession number 1-2621.

12. Use of a nucleic acid probe specifically hybridizing under highly stringent conditions with the nucleic acid sequence of SEQ ID No: 1, to determine the presence or absence of the virulence gene coding for the protein as claimed in claim 1, in a biological sample.

13. A nucleotide primer, **characterized in that** it allows specific amplification of at least a fragment of the sequence SEQ ID No: 1, starting from cells infected with Leishmania major, thus allowing the presence or absence of the virulence gene coding for the protein of SEQ ID NO: 2 (LmPDI) to be determined in a biological sample.

14. A purified antibody, specifically recognizing the protein as claimed in claim 1.

15. An immunogenic composition comprising a protein as claimed in claim 1, 2 or 5 and/or a recombinant polypeptide as claimed in claim 3 or claim 4, and/or a recombinant nucleic acid the sequence for which codes for such a protein, and/or a recombinant nucleic acid the sequence of which codes for such a polypeptide, and/or a nucleic acid sequence as claimed in claim 7, and/or a vector for a nucleic acid comprising such a nucleic acid, and/or a cell comprising such a vector, said immunogenic composition being capable of in vitro stimulation of the proliferation of mononuclear cells deriving from individuals who have come into contact with a Leishmania parasite.

16. The immunogenic composition as claimed in claim 15, capable of in vitro stimulation of the proliferation of mononuclear cells deriving from individuals who have come into contact with Leishmania major.

17. The immunogenic composition as claimed in claim 15 or claim 16, having a pharmaceutically acceptable formulation for administration to a human or animal host.

18. The immunogenic composition as claimed in any one of claims 15 to 17, capable of inducing a type Th1 immune response when administered to a human or animal host.

19. A vaccinating composition comprising a protein as claimed in claim 1, 2 or 5 and/or a recombinant polypeptide as claimed in claim 3 or claim 4, and/or a recombinant nucleic acid the sequence for which codes for such a protein, and/or a recombinant nucleic acid the sequence of which codes for such a polypeptide, and/or a nucleic acid sequence as claimed in claim 7, and/or a vector for a nucleic acid comprising such a nucleic acid, and/or a cell comprising such a vector, said vaccinating composition being intended to protect a human or animal host against leishmaniasis.

20. The vaccinating composition as claimed in claim 19, having a pharmaceutically acceptable formulation for administration to a human or animal host.

21. The immunogenic and/or vaccinating composition as claimed in any one of claims 15 to 20, further comprising an antigen foreign to Leishmania and/or a nucleic acid sequence coding for an antigen foreign to Leishmania.

22. A method for screening molecules that are susceptible of inhibiting the growth of Leishmania major, comprising a step for evaluating the capacity of said molecules to inhibit the activity of the protein as claimed in claim 1.

23. A screening method as claimed in claim 22, in which the step for evaluating the capacity of a molecule to inhibit the activity of the protein as claimed in claim 1 is carried out in a test for reactivating reduced and denatured RNase A comprising the following steps:
• incubating reduced and denatured RNase A in the presence of the protein as claimed in claim 1 under conditions allowing its reactivation;
• incubating reduced and denatured RNase A under conditions identical to those allowing its reactivation by the protein as claimed in claim 1, by adding the molecule to be tested;
• comparing the results obtained in the absence and in the presence of the test molecule, a fault in the reactivation of RNase A in the presence of the test molecule revealing that said molecule has an inhibiting activity in respect of the protein as claimed in claim 1.

24. The screening method as claimed in claim 22 or claim 23, further comprising a test for inhibiting the growth of Leishmania major in a liquid medium.

25. Use of one or more compounds which inhibit a Leishmania major protein disulfide-isomerase (PDI), for the preparation of a pharmaceutical composition intended for prophylaxis, attenuation or treatment of infection with Leishmania major.

26. Use as claimed in claim 25, in which said inhibitor compound or one of said inhibitor compounds is an anti-PDI or anti-LmPDI antibody, bacitracin, zinc bacitracin, 5,5'-dithiobis(2-nitrobenzoic) acid (DTNB), p-chloromercuribenzene sulfonic acid (pCMBS) or tocinoic acid.

27. Use as claimed in claim 25 or claim 26, for the preparation of a composition for topical, oral or parenteral administration to a human or animal host.

28. Use of bacitracin or zinc bacitracin for the preparation of a pharmaceutical composition for inhibiting the growth of a parasite responsible for leishmaniasis or as an active agent against a leishmaniasis infection.

29. A pharmaceutical composition intended for the treatment of a leishmaniasis infection, comprising an antibody as claimed in claim 14.

30. The composition as claimed in claim 29, suitable for topical, oral or parenteral administration.

31. A pharmaceutical composition intended for the treatment of an infection with Leishmania, containing one or more protein disulfide-isomerase (PDI) inhibitors.

32. A pharmaceutical composition as claimed in claim 31, containing bacitracin or zinc bacitracin.

33. An in vitro method for diagnosing an infection by a parasite responsible for leishmaniasis, **characterized in that** it comprises:
• bringing at least one antibody as claimed in claim 14 into contact with a biological sample from a subject partially infected with a parasite responsible for leishmaniasis under conditions allowing the formation of an immune complex between said antibody and antigenic proteins contained in the sample;
• detecting said complex.

34. A diagnostic kit for carrying out the method as claimed in claim 33, **characterized in that** it comprises:
• at least one antibody as claimed in claim 14;
• a medium suitable for forming an immune complex with said antibody;
• reagents allowing the detection of any complexes that are formed;
• control samples, if appropriate.
